(19) Europäisches Patentamt
European Patent Office
Office européen des brevets

(11) **EP 2 155 252 B1**

(12) **EUROPEAN PATENT SPECIFICATION**

(45) Date of publication and mention
of the grant of the patent:
**04.12.2013 Bulletin 2013/49**

(21) Application number: **08772791.3**

(22) Date of filing: **12.06.2008**

(51) Int Cl.:
*A61K 47/36* (2006.01)     *A61K 47/08* (2006.01)
*A61K 47/12* (2006.01)     *A61K 47/24* (2006.01)
*A61K 9/00* (2006.01)      *A61P 35/00* (2006.01)
*A61K 31/337* (2006.01)

(86) International application number:
**PCT/CA2008/001128**

(87) International publication number:
**WO 2008/151433 (18.12.2008 Gazette 2008/51)**

(54) **INJECTABLE POLYMER-LIPID BLEND FOR LOCALIZED DRUG DELIVERY**

INJIZIERBARE POLYMER-LIPID-MISCHUNG ZUR LOKALISIERTEN ARZNEIMITTELABGABE

MÉLANGE POLYMÈRE-LIPIDE INJECTABLE PERMETTANT UNE DÉLIVRANCE LOCALISÉE DE MÉDICAMENT

(84) Designated Contracting States:
**AT BE BG CH CY CZ DE DK EE ES FI FR GB GR HR HU IE IS IT LI LT LU LV MC MT NL NO PL PT RO SE SI SK TR**

(30) Priority: **12.06.2007 US 943532 P**

(43) Date of publication of application:
**24.02.2010 Bulletin 2010/08**

(73) Proprietors:
• **Allen, Christine**
  **Toronto, ON M4V 1B9 (CA)**
• **Grant, Justin**
  **Toronto, ON M5R 1G1 (CA)**
• **Cho, Jaepyoung**
  **Toronto, ON M2J 3J7 (CA)**
• **Lim Soo, Patrick**
  **Toronto, ON M5S 3A3 (CA)**
• **Piquette-Miller, Micheline**
  **Toronto ON M4G 4K5 (CA)**

(72) Inventors:
• **Allen, Christine**
  **Toronto, ON M4V 1B9 (CA)**
• **Grant, Justin**
  **Toronto, ON M5R 1G1 (CA)**
• **Cho, Jaepyoung**
  **Toronto, ON M2J 3J7 (CA)**
• **Lim Soo, Patrick**
  **Toronto, ON M5S 3A3 (CA)**
• **Piquette-Miller, Micheline**
  **Toronto ON M4G 4K5 (CA)**

(74) Representative: **Lee, Nicholas John et al**
**Kilburn & Strode LLP**
**20 Red Lion Street**
**London WC1R 4PJ (GB)**

(56) References cited:
**WO-A1-2005/087221**

• **GRANT J ET AL: "Chapter 10: Chitosan as a biomaterial for preparation of depot-based delivery systems" WATER-SOLUBLE POLYMERS: SYNTHESIS, SOLUTION PROPERTIES AND APPLICATIONS, AMERICAN CHEMICAL SOCIETY, WASHINGTON, DC, US, no. 228th conf, 1 January 2006 (2006-01-01), pages 201-225, XP009094801 ISBN: 978-0-541-23408-9**
• **LE TIEN C. ET AL.: 'N-acylated chitosan: hydrophobic matrices for controlled drug release' JOURNAL OF CONTROLLED RELEASE vol. 93, 2003, pages 1 - 13, XP004471359**
• **CHO J. ET AL.: 'Synthesis and physiochemical and dynamic mechanical properties of a water-soluble chitosan derivative as biomaterial' BIOMACROMOLECULES vol. 7, 2006, pages 2845 - 2855, XP008125136**
• **GRANT J. ET AL.: 'Influence of molecular organization and interactions on drug release for an injectable polymer-lipid blend' INTERNATIONAL JOURNAL OF PHARMACEUTICS vol. 360, August 2008, pages 83 - 90, XP022853130**

## Description

### Technical Field

The present invention relates in general to biodegradable, biocompatible systems for localized drug delivery. The present invention relates more particularly to injectable polymer-lipid blend systems for localized drug delivery. compatible

### Background Art

When administered in standard intravenous or oral formulations, many pharmaceutical agents fail to reach the target organ in an effective concentration, or are not effective due to rapid elimination. This lack of effectiveness of the drug can result from a number of factors including: acid hydrolysis or incomplete absorption from the gastrointestinal tract, inability of the drug to cross physiological membranes such as the blood brain barrier, insufficient distribution to the site of action, enzymatic deactivation of the compound in the liver or blood prior to reaching the target organ, and rapid secretion of the drug into bile, urine or feces.

Delivery of drugs directly to the site of action using localized delivery systems provides advantages in that it provides high drug concentrations at the site of action while reducing systemic exposure. Indeed, in recent years it has been demonstrated that local administration offers increased efficacy and decreased toxicity of anti-neoplastic agents in the treatment of various cancers.

Material blends have been employed in the preparation of advanced drug delivery systems by combining the favorable properties of excipients to produce hybrid composites with enhanced characteristics. In recent years, injectable blends in the form of pastes and gels have been prepared which allow for easy administration directly to a targeted site and avoid prevalent toxicities that occur via systemic administration. Furthermore, sustained release of drug from a blend formulation over an extended period can increase the overall efficacy of the drug by several means including: higher drug content at the treatment site, prolonged exposure for cell cycle specific drugs, increased tumor penetration, reduced drug resistance and improved patient compliance.

Studies have shown that injectable blend systems are effective for localized delivery of anticancer agents, growth factors, proteins, antisense oligonucleotides, and scaffolds.

The development of biocompatible, biodegradable injectable systems that can be administered in liquid form and remain as a semi-solid depot system over an extended period of time are clinically desirable. The formation of a semi-solid implant allows for a targeted localized drug delivery system that may be more effective than current medical therapies. For example, localized drug delivery devices may require a lower drug dosage to achieve therapeutic concentrations as the drug is released at a specific site which in turn will reduce the prevalence of systemic side effects. Furthermore, its ease of application by syringe injection may replace surgical procedures required for implanting medical devices. Patients with cancer, particularly solid tumors, diabetes, addiction disorders and wounds are ideal candidates for this drug therapy approach.

To date, mostly synthetic polymers have been used to develop injectable semi-solid depot systems which may be characterized into four distinct groups: in situ precipitation, thermoplastic pastes, in situ cross-linked systems and in situ solidifying organogels. In situ precipitation is a method in which the injectable system solidifies in the body by precipitation of the material due to environmental changes such as temperature, pH, and solvent removal. For example, water insoluble polymers such as poly(lactide) (PLA), poly(lactide-co-glycolide (PLGA), and poly(lactide)-co-poly(caprolactone) (PLA-co-PCL) may be dissolved in a physiologically compatible water-miscible solvent and injected into an aqueous environment to precipitate the polymer. The polymer concentration, molecular weight, type of polymer and solvent and the addition of surfactants have been shown to influence both the rate of precipitation and drug release from these systems. For thermoplastic pastes, polyesters such as PLA, PCL, or PLGA may be injected into the body as a melt and solidify upon cooling to body temperature of 37°C. These polymers also contain a low intrinsic viscosity below 0.8 dl/g in order to be easily injected via 22 gauge syringe. However, high temperatures are often required for thermoplastic pastes at the time of injection and the degradation of polyester-based materials produces acid byproducts that may compromise the biocompatibility of the implant. Therefore, there is a need for new biomaterials to be employed in the preparation of implantable drug delivery systems.

Chitosan-based polymer blends have been found to be useful for controlled drug delivery because they degrade uniformly into non-toxic molecules that are non-mutagenic, non-cytotoxic, and non-inflammatory. Chitosan is a natural, biodegradable cationic polysaccharide, which has previously been described as a promoter of wound healing. Chitosan is a commercially available inexpensive polymer which is mainly composed of D-glucosamine units that are generated through catalyzed N-deacetylation of chitin, a natural material extracted from fungi, shellfish exoskeletons and algae. Chitosan has excellent tissue compatibility and biodegradability which renders it ideal for injection.

Various acids, such as lactic acid, acetic acid and hydrochloric acid may be used to dissolve chitosan to formulate various drug delivery systems. Following preparation, however, residual acid may remain within the drug formulation, thereby reducing the biocompatibility or non-toxicity of the delivery system, as well as accelerating the degradation of some drugs.

To avoid the use of acids, water soluble chitosan can be synthesized. According to one known method, chitosan may be conjugated with glycidyltrimethylammonium chloride ("GTMAC") in order to increase the biopolymer's water solubility. (Cho, J., et al., Biomacromolecules, 2006, 7(10), 2845-2855.) This chitosan derivative has high water solubility, up to 25 g/dL, without use of any acid. Water soluble chitosan ("WSC") is known to have antimicrobial activity and the quaternary ammonium group in GTMAC is also found to inhibit the proliferation of various cancer cell lines. The water solubility and biological properties of WSC grants an excellent polymer candidate, which can be used to formulate drug delivery systems, and serve as a polymer matrix for the drug to provide a controlled release drug delivery platform. Furthermore, its biodegradability allows the delivery system to completely degrade *in vivo.*

Fatty acids ("FA") are known to be involved in a number of biological processes, including metabolism, energy storage, immune response, blood clotting, blood pressure regulation, formation of biological structures such as membranes, and formation in the body of compounds such as prostaglandins. Fatty acids are currently approved for use as inactive excipients in sublingual, oral, topical and vaginal formulations in the form of films, capsules, sprays, ointments, emulsions and creams. N-acetlyated chitosan derivatives have been prepared using fatty acid chlorides to produce a hydrophobic matrix in tablets for the sustained release of acetaminophen. (Le Tien, C., et al., Journal of Controlled Release, 2003. 93(1): p. 1-13.) In this prior art case, however, the initial chitosan component is achieved by dissolving it in acetic acid to ensure total solubility of the chitosan derivative, which is then modified using fatty acid chlorides. The use of an acidic solution to prepare the derivative can cause toxicity and drug degradation in the blend.

Recently, researchers have exploited non-covalent or physical cross-linking of chitosan polymer chains to achieve electrostatic and hydrogen bonding, which increases the stability and biocompatibility of the hydrogel. For example, negatively charged molecules, such as oligonucleotides DNA and RNA, engage in electrostatic interaction with chitosan to produce adducts which are stable for up to 15 days (U.S. Patent Publication No. 2003/0134810 to Springate et al.). In addition, Hoffman et al. have developed a cross-linked chitosan-glycerol film for the mucosal delivery of glycoproteins (Hoffman et. al, J. Control. Rel. (2001) 72:35-46).

As well, U.S. Patent No. 7,060,285 to Muller ("Muller") describes use of an oil-in-water emulsion for poorly soluble active agents. The invention in Muller focuses mainly on an emulsion for intravenous administration. The approach disclosed is limited in terms of drug concentration as particular agents may have relatively low solubility in the emulsion. Furthermore, the invention in Muller requires relatively high energy or high pressure homogenization to prepare the emulsion, which is both complex and time consuming. Further, it is known that emulsions generally are unstable.

Phospholipids, due to their excellent biocompatibility, have been commonly used in preparation of drug delivery systems in the form of liposomes, emulsions, and films. Phosphatidylcholine lipids are the major membrane phospholipids found in eukaryotic cells. Egg phosphatidylcholine ("ePC") consists of a mixture of phosphatidylcholine lipids having hydrocarbon chains of different lengths and degrees of saturation. Pure ePC includes 34% (w/w) dipalmitoylphosphatylcholine (DPPC, 16:0, $T_M$=41°C) and 65% (w/w) dioleoylphosphatylcholine (DOPC, 18:1, $T_M$=-20°C), 18% (w/w) dilinoeoylphosphatidylcholine (DLPC, 18:2, $T_M$=-53°C) 11% (w/w) distearoylphosphatidylcholine (DSPC, 18:0, $T_M$=55°C) and a small amount of diarachidonoylphosphatidylcholine (DAPC, 20:4, $T_M$=-70°C).

A blend prepared from two distinct families of materials, namely the biopolymer chitosan and the lipid ePC ("PoLi"), for use as an implantable film for localized drug delivery has been disclosed in United States Patent Application No. 2005/0208122 to Allen et al. The implant provides a sustained release of a hydrophobic drug over an extended period in physiological solutions and *in vivo*. In the PoLi system, acid is used to dissolve chitosan. Although the PoLi implant system demonstrates both biocompatibility and efficacy *in vitro* and *in vivo*, it requires a long processing time and surgical implantation at a tumour resection site to provide controlled release of an anticancer agent or other drug.

In light of the foregoing, an injectable biodegradable, biocompatible controlled drug delivery system comprising a chitosan based material in combination with a fatty acid and ePC, and the use of this system in the delivery of pharmaceutically active agents, are desirable. It would also be desirable if said injectable system could be prepared without the use of an acid for dissolving the chitosan based material.

## Dislcosure of Invention

The present invention relates to an injectable composition comprising a semi-solid blend of:

    (a) a chitosan based material;
    (b) a fatty acid or a fatty acid chloride or a fatty aldehyde having an acyl chain length of C8-C16;
    (c) a phospholipid; and
    (d) at least one pharmaceutically active agent.

The present invention therefore relates to an injectable drug delivery composition for the controlled release of a pharmaceutically active agent, the injectable drug delivery composition comprising a semi-solid blend of at least three carrier materials which have favorable properties for drug delivery: a chitosan-based material; one or more fatty acids having an acyl chain length of C8-C16, preferably C10-C14, most preferably C12; and one or more phospholipids, loaded with at least one pharmaceutically active agent. The fatty acid may be a fatty acid chloride or a fatty acid aldehyde, preferably laurinaldehyde. The composition is for the delivery of at least one pharmaceutically active agent to provide controlled release when administered to a target site in a mammal.

According to the present invention, the blend can include a chitosan derivative which is water soluble chitosan. The water soluble chitosan may be produced by conjugating a chitosan material with GTMAC, for example. Other forms of water soluble chitosan are also within the scope of the present invention.

According to the present invention the viscosity of the drug delivery blend is related to the fatty acid selected and the pharmaceutically active agent. The size of the acyl chain of the fatty acid is therefore selected to achieve an appropriate viscosity for injectability.

According to the invention, the phospholipid may be selected from the group consisting of phosphatidylcholine, phosphatidylethanolamine, egg phosphatidylcholine and phosphatidylglycerol. The pharmaceutically active agent may be selected from the group consisting of carmustine, methotrexate, carboplatin, cisplatin, oxaliplatin, 5-fluorouracil, 5-fluorouridine, cytarabine, leuprolide acetate, cyclophosphamide, vinorelbine, pilocarpine, paclitaxel, mitomycin, camptothecin, doxorubicin, daunorubicin and docetaxel. Alternatively, the pharmaceutically active agent may be an oligonucleotide, a peptide or a protein.

According to the invention, the injectable drug delivery composition may be formulated for injection through a syringe needle.

The present invention further relates to a drug delivery platform for the loading, delivery and controlled release of a pharmaceutically active agent, comprising a water soluble chitosan; a fatty acid having an acyl chain length of C12; and a phospholipid selected from the group consisting of phosphatidylcholines, phosphatidylethanolamine, ePC and phosphatidylglycan

The present invention further provides a method of manufacturing a drug delivery composition for controlled release in a mammal comprising preparing a water soluble chitosan from a chitosan-based material; and forming a complex of the water soluble chitosan with a fatty acid and a phospholipid to form an injectable material for providing the controllable release of at least one pharmaceutically active agent. Water soluble chitosan may be prepared, for example, by conjugating a chitosan-based material with GTMAC.

There is described a method of treating, preventing or inhibiting disease comprising administering a therapeutically effective amount of an injectable drug delivery composition for the controlled release of a pharmaceutically active agent. The disease may be any disease which is treatable at localized areas of the body, for example, a cancer.

There is further described the use of a chitosan-based material, a fatty acid, a phospholipid, and at least one pharmaceutically active agent in the manufacture of a drug delivery system to treat disease.

An object of one aspect of one embodiment of the present invention is to provide improved injectable polymer-lipid blends of pharmaceutically active agents as a method to provide sustained, local delivery of individual drugs or drug combinations.

Advantages of the present invention include the protection of drugs from degradation, the maintenance of effective concentrations of drugs, a decrease in the frequency of administration of drugs, a decrease in the dosage of therapeutic agents administered to patients, and a decrease in toxicities or side-effects that may result following systemic administration of drugs.

**Brief Description of the Drawings**

A detailed description of the preferred embodiments is provided herein below by way of example only and with reference to the following drawings, in which:

Fig. 1 illustrates the chemical structures of components of possible drug delivery blends in accordance with one embodiment of the present invention. The letter 'm' represents an acyl chain length of from C10 to C16. Where X is Cl, the fatty acid is a fatty acid chloride. Where X is H, the fatty acid is a fatty aldehyde.

Fig. 2 illustrates turbidity of 1:4:1 (w/w/w) WSC-FA-ePC blend formulations differing in FA acyl chain length (n = 3, in 0.01 M PBS, pH = 7.4).

Fig. 3 illustrates (a) DSC thermograms of WSC, ePC and C12 FA (b) WSC-FA blends (1:1, w/w) with varying FA acyl chain length; and (c) WSC-FA-ePC blends (1:4:1, w/w/w) with varying FA acyl chain length.

Fig. 4 illustrates Fourier transform infra-red spectra of (a) WSC, lauroyl chloride (C12 FA), WSC-FA(C12) blend, WSC-ePC blend and 1:4:1 (w/w/w) WSC-FA-ePC, and (b) 1:4:1 (w/w/w) WSC-FA-ePC blends varying in FA acyl chain length from C10 to C16.

Fig. 5 illustrates optical micrographs of the WSC:FA(C12):ePC blends (a) 1:0:1 (w/w/w) (b) 1:4:0 (w/w/w) and (c) 1:4:1 (w/w/w).

Fig. 6 illustrates fluorescence confocal microscopy of 1:4:1 (w/w/w) WSC-FA-ePC blends containing (a) C10 FA, (b) C12 FA, and (c) C16 FA where regions of lipid and/or fatty acid are in the top left image, WSC is in the top right image and the overlay of the WSC and FA-ePC regions is in the bottom left.

Fig. 7 illustrates a comparison of viscosity as a function of shear stress for WSC-FA-ePC blends that vary in FA acyl chain length for (a) blend ratio WSC: FA: ePC = 1:2:1 (w/w/w) and (b) blend ratio WSC: FA: ePC = 1:4:1 (w/w/w).

Fig. 8 illustrates percent cumulative release of carbon-14 labeled paclitaxel from 1:4:1 (w/w/w) WSC-FA-ePC blend formulations (in 0.01 M phosphate buffer solution containing 0.2% lysozyme at 37°C, pH = 7.4, n=3.

Fig. 9 illustrates the drug release profile of (a) the WSC-FA C12-ePC 1:4:1 (w/w/w) blend fitted to different kinetic models and (b) the 1:4:1 (w/w/w) WSC-FA-ePC blends varying in FA acyl chain length modeled by the Peppas-Sahlin equation.

Fig. 10 illustrates the ratio of Case II transport to Fickian diffusion ($K_{P\_S,2}t^m / K_{P\_S,1}$) as a function of release time for the 1:4:1 (w/w/w) WSC-FA-ePC blends varying in FA acyl chain length.

Fig. 11 illustrates cell viability for blend compositions in which the FA is lauroyl chloride.

Fig. 12 illustrates turbidity data for blend compositions in which the FA is laurinaldehyde.

Fig. 13 illustrates turbidity data for additional blend compositions in which the FA is laurinaldehyde.

Fig. 14 illustrates partition coefficients for high and low molecular weight chitosan compositions.

Fig. 15 illustrates cumulative release of paclitaxel from a blend composition.

Fig. 16 illustrates experimental data and data predicted by the Ritger-Peppas and Peppas-Sahlin models for paclitaxel release.

Fig. 17 illustrates cell viability for blend compositions including laurinaldehyde.

Fig. 18 illustrates a comparison of docetaxel release from fatty acid chloride and laurinaldehyde blend compositions.

Figs. 19A and 19B illustrate release of varying loads of docetaxel from laurinaldehyde blend compositions.

Figure 20 illustrates plasma alanine aminotransferase (ALT) levels in CD-1 mice between 2 days and 4 weeks after injection of drug-free or docetaxel-loaded blend compositions.

Figure 21 illustrates average plasma concentration levels of docetaxel ($\mu$g/mL) in CD-1 mice at times between 2 days and 7 weeks after injection of docetaxel-loaded blend compositions.

In the drawings, preferred embodiments of the invention are illustrated by way of example. It is to be expressly understood that the description and drawings are only for the purpose of illustration and as an aid to understanding, and are not intended as a definition of the limits of the invention.

## Best Mode(s) for Carrying out the Invention

### Definitions

The following paragraphs provide definitions of the terms used herein. All terms used herein including those specifically discussed below in this section, are used in accordance with their ordinary meanings unless the context or definition clearly indicates otherwise. Also unless indicated otherwise except within the claims the use of "or" includes "and" and vice-versa. Non-limiting terms are not to be construed as limiting unless expressly stated or the context clearly indicates otherwise (for example, "including", "having" and "comprising" typically indicate "including without limitation"). Singular forms included in the claims such as "a", "an" and "the" include the plural reference unless expressly stated or the context clearly indicates otherwise.

"Polymer" indicates a molecule composed of a number of identical repeating units.

"Chitosan" includes various derivatives of chitosan such as carboxymethylchitosan, oleoyl chitosan and pegylated chitosan (Carbomer, Inc., Westborough, Mass.)

"Composition" as used herein should be understood to indicate a combination of multiple substances into an aggregate mixture.

"Controlled release" indicates the release of therapeutically active agents or pharmaceutically active agents into the surrounding media or body over an extended time period. The time period may be from approximately a few hours to several months.

"Drug", "therapeutic agent" and "therapeutic" each indicates any molecule that has a significant effect on the body to treat or prevent conditions or diseases.

"Fatty acid" ("FA") includes, without limitation, fatty acid chloride and fatty aldehyde of any acyl chain length.

"Pharmaceutically active agent" means any of a drug, a therapeutic agent, a pro-drug or a diagnostic.

"Hydrophobic Drug" means any pharmaceutically active agent that is only soluble in water at less than 50 mg/L at 25°C.

"Anti-proliferative agent" means a molecule that acts to inhibit proliferative events. Examples of anti-proliferative agents include, but are not limited to, paclitaxel, carboplatin, cisplatin.

### Detailed Description

In order to achieve delayed release and effective sustained concentrations of a particular drug in proximity to a target organ, the drug may be combined with a carrier or vehicle that is biocompatible and biodegradable. Suitable carriers for drug incorporation range in size from small molecules to macromolecules, including high molecular weight polymers. Polymer-based devices can be used to release a drug at a specific location at a controlled release rate over an extended period of time. The most desirable polymeric means for drug delivery is one that is inexpensive, biocompatible, and biodegradable, and provides uniform, controlled release of the drug in an aqueous environment.

The polymer-lipid blend of the present invention is a local drug delivery device that is applied to a targeted site and allows prolonged controlled release of a drug or other active agent over an extended period, which may range from several days to several months. The blend can be administered in a variety of ways, but is particularly suited as an injectable drug delivery means.

In accordance with one embodiment of the present invention there is provided an injectable drug delivery composition for the controlled release of a pharmaceutically active agent, the injectable drug delivery composition comprising a semi-solid blend of at least three carrier materials which have favorable properties for drug delivery: a chitosan-based material; one or more fatty acids having an acyl chain length of C8-C16, preferably C10-C14, most preferably C12; and one or more phospholipids, loaded with at least one pharmaceutically active agent. The fatty acid may be a fatty acid chloride or a fatty acid aldehyde, preferably laurinaldehyde. The composition is for the delivery of at least one pharmaceutically active agent to provide controlled release when administered to a target site in a mammal. The unique formulation provides controlled release of hydrophilic agents, hydrophobic agents or combinations of hydrophilic and hydrophobic agents.

Chitosan is a linear polysaccharide composed of two monosaccharides linked by glycoside bonds and is manufactured by deacylation of chitin. Chitosan is a mucoadhesive, biocompatible polymer that is commercially available in a range of molecular weights and degrees of deacylation. As the molecule has a protonable primary amine on a side chain, chitosan has weak cationic properties. Chitosan is a naturally occurring biodegradable, biocompatible polysaccharide that has been investigated for use in a variety of biomedical applications including wound dressings, sutures, artificial skin, tissue engineering and drug delivery.

Chitosan is typically not soluble in water but may be dissolved in weak acids such as a 2% acetic acid solution, and the chitosan degrades in vivo under the action of enzymes such as lysozymes. Various acids (i.e. lactic acid, acetic acid and hydrochloric acid) are normally used to dissolve chitosan in order to formulate various drug delivery systems.

Unfortunately, following preparation, residual acid may remain within the drug formulation and in turn reduce the biocompatibility or non-toxicity of the delivery system as well as accelerate the degradation of some drugs.

According to a particular aspect of the present invention, to avoid the use of acids, chitosan can be conjugated with glycidyltrimethylammonium chloride ("GTMAC") in order to increase the biopolymer's water solubility. This chitosan derivative, referred to herein as water soluble chitosan, or "WSC", has high water solubility of up to 25 g/dL without the use of any acid. WSC is also known to have antimicrobial activity and the quaternary ammonium group in GTMAC has been found to inhibit the proliferation of various cancer cell lines. WSC also acts as a surfactant for the fatty acid component as it prevents phase separation that occurs when the fatty acid is in contact with distilled water. Furthermore, WSC's biodegradability allows the delivery system to completely degrade *in vivo.*

Fatty acids are known to be involved in a number of biological processes and in forming biological structures and compounds within the body. In accordance with a particular aspect of the present invention, a fatty acid may be mixed with WSC and phospholipids to develop a stable injectable blend for drug delivery. The fatty acids used in the blend may include, without limitation, fatty acid chlorides or fatty aldehydes such as laurinaldehyde. The fatty acid acyl chain length may vary, for example, from C8 to C16.

The phospholipid or lipid components may include phosphatidylcholines, phosphatidylserines, phosphatidylinositols, phosphatidylethanolamines, phosphatidylglycerols, or a mixture thereof. The source of phospholipids may be a commercially available egg yolk extraction primarily comprised of phosphatidylcholine (>60%) and other phospholipids (40%). Phosphatidylcholine is the principle membrane phospholipid found in human and animal cells and is commonly used in pharmaceutical liposome formulations.

The pharmaceutically active agents of the present invention can be any of those agents which are generally required to be frequently administered in order to maintain an effective blood concentration or an effective concentration of the pharmaceutically active agent content locally.

As an example, pharmaceutically active agents may include various anticancer or anti-proliferative agents, including, without limitation, carmustine, methotrexate, carboplatin, cisplatin, oxaliplatin, 5-fluorouracil, 5-fluorouridine, cytarabine, leuprolide acetate, cyclophosphamide, vinorelbine, pilocarpine, paclitaxel, mitomycin, camptothecin, doxorubicin, and daunorubicin. Other examples of pharmaceutically active agents include oligonucleotides, peptides and proteins.

The combination of the WSC and ePC forms a partially miscible blend that is stabilized by the addition of FA. In accordance with another aspect of the present invention, the stability of polymer-lipid blends is known to be related to the ratio of the components and the acyl chain length of the fatty acid employed. In particular, the effect of fatty acyl chain lengths, ranging from C6-C16, is to alter the viscosity of the final blend. Generally, as the hydrocarbon chain length of the fatty acid increases, the viscosity and yield stress values increase. The rheological properties of injectable systems are important for drug formulations as a blend with low viscosity may not exhibit a controlled drug release profile. If the blend is too viscous however, difficulties may be encountered when injecting the formulation. In general, the viscosity in a polymeric system increases when there are more interactions between macromolecules, such as entanglement, physical interaction (i.e. van der waals, hydrophobic and hydrogen bonding interactions) and cross-linking. These interactions between macromolecules can be used to trap the drug in a polymer matrix. Thus, the more interactions present in a polymer system, the slower the rate of drug release.

The solubility of the drug in a formulation can also strongly influence the drug release performance. Accordingly, depending on the specific treatment that is required, the drug release can be controlled by modifying either the type of fatty acid used or the specific concentrations of each of the individual components in the blend.

Thus the concentration of the three components and the length of the fatty acid acyl chain employed have a significant impact on the development of an injectable blend formulation. A C12 fatty acid blend is an optimal chain length for preparation of a stable semi-solid injectable blend system for paclitaxel ("PTX"). Furthermore, PTX release from the blend systems shows Fickian diffusion for C12 FA and C14 FA blends. The C16 FA blend, however, involves Case II transport mechanisms.

As indicated in Table 1, in which O = stable, Δ = partial phase separation, and X= complete phase separation, an increase in the blend of the proportion of any of fatty acid, ePC or chitosan, or in the molecular weight of chitosan, results in an increase in stability.

Table 1. Stability of WSC-FA-ePC blends in 0.01 M PBS

| Formulation | WSC:FA:ePC (w/w/w) | Stability at RT after injection into PBS | Stability after 72 hrs at 37°C in PBS |
|---|---|---|---|
| C12 Cl (high MW chitosan) | | | |
| 1 | 0.5 : 4 : 1 | Δ | Δ |

(continued)

| Formulation | WSC:FA:ePC (w/w/w) | Stability at RT after injection into PBS | Stability after 72 hrs at 37°C in PBS |
|---|---|---|---|
| C12 Cl (high MW chitosan) | | | |
| 2 | 1 : 4 : 1 | O | O |
| 3 | 1.5 : 4 : 1 | O | O |
| 4 | 2 : 4 : 1 | O | X |
| 5 | 1 : 1 : 1 | X | X |
| 6 | 1 : 2 : 1 | O | Δ |
| 7 | 1 : 3 : 1 | O | Δ |
| 8 | 1 : 4 : 0.5 | O | O |
| 9 | 1 : 4 : 1.5 | O | O |
| 10 | 1 : 4 : 2 | O | O |
| C12 CHO (low MW chitosan) | | | |
| 11 | 1 : 1 : 1 | X | X |
| 12 | 1 : 2 : 1 | X | X |
| 13 | 1 : 3 : 1 | O | O |
| 14 | 1 : 4 : 1 | O | O |

Conveniently, the compositions of the present invention can be prepared as injectable drug delivery systems for intraperitoneal, intraarticular, intraocular, intratumoral, perivascular, subcutaneous, intracranial, intramuscular, intravenous, periophthalmic, inside the eyelid, intraortal, intranasal, intrabladder, intravaginal, intraurethral, or intrarectal delivery. Preferably, the compositions can be formulated to be injected through a syringe needle, though mode of administration need not be limited to injection. The subject or patient can be a human or other mammal.

Advantages of the present invention should be understood to include: protection of therapeutic agents from degradation; maintenance of effective concentrations of the therapeutic either locally or systemically, decrease of the frequency of administration of the therapeutic agent; decrease of the amount of therapeutics administered to patients per dose; and decrease of the toxicities or side effects that usually result following systemic administration.

In accordance with other embodiments of the present invention there are provided methods of manufacturing a controlled release drug delivery composition comprising a chitosan-based material, a fatty acid, at least one phospholipid component and at least one pharmaceutically active agent to provide controllable release of the at least one pharmaceutically active agent when administered to a mammalian subject or patient. The methods of manufacture may produce a solution having desirable viscosity for use as a stable semi-solid injectable blend.

According to one embodiment, the preparation of a blend may include an initial step of modifying chitosan, chitin or a mixture thereof to a WSC derivative using GTMAC. This is achieved by generating a mixing GTMAC and chitosan in a ratio of 3:1 mol/mol, for example, following by a suitable purification procedure. The WSC derivative is then weighed and dissolved in distilled water. The ePC is then solubilized with fatty acids (varying in acyl chain length) and added to the WSC solutions in specific weight ratios. WSC acts as a surfactant for FA as it prevents phase separation that occurs when FA is in contact with distilled water. Lastly, the WSC-FA-ePC blend may be vortexed, e.g., for two minutes, and stored at room temperature.

There are described methods of treating or inhibiting a disease comprising administering to a patient a therapeutically effective amount of a composition described herein. The injectable delivery system of the present invention may be advantageous as a treatment strategy for various diseases, including cancers of the prostate, breast, ovarian, bladder, brain, liver, gastric, head and neck.

The drug delivery composition of the present invention affords a method of delivering by controlled release at least one pharmaceutically active agent. As outlined herein, there are various cancers in which the current invention could be utilized. The current invention could also be utilized as a drug delivery system in the treatment of diseases other than cancer, particularly where a localized method of drug delivery is optimal for favourable health outcomes.

The present invention will be further understood by reference to the following non-limiting examples.

EXAMPLE 1

Preparation and Analysis of Injectable System

In a study, FA varying in chain length from C8 to C16 were mixed with WSC and ePC to develop a stable injectable blend for drug delivery. Thermal analysis was used to determine the stability of the blend components at body temperature. FTIR measurements were employed to investigate the interactions present between the components in order to optimize the stability of the blend. Morphological examination provided an indication of the functionality of each of the components within the formulation at a microscale level. Also, to determine the optimal blend for use as an injectable formulation, rheological and stability measurements were required. Lastly, the release of the anticancer agent PTX from the WSC-FA-ePC blends was evaluated in physiological solution. Relationships between the composition of the WSC-FA-ePC blend and its properties were identified in order to optimize the performance of the blends for pharmaceutical applications.

Materials

Chitosan (90%) was purchased from Marinard Biotech Inc. (Rivière-au-Renard, QC, Canada). ePC (>99%) was obtained from Northern Lipids Inc. (Vancouver, BC, Canada). Unlabelled PTX (>99%) and $C^{14}$-PTX were purchased from Hande Tech Development Co. (Houston, TX) and Moravek (Brea, CA), respectively. The fluorescent probe 3-phosphoethanolamine-N-(7-nitro-2-1,3-benzoxadiazol-4-yl) (NBD-DPPE) was purchased from Avanti Polar Lipids Inc. (Alabaster, AL). Glycidyltrimethylammonium chloride (GTMAC), acetone, ethanol, methanol, acetic acid (AcOH), fatty acid chlorides (i.e. capryloyl chloride (C8), decanoyl chloride (C10), lauroyl chloride (C12), myristoyl chloride (C14) and palmitoyl chloride (C16)) and all other chemicals were purchased from Sigma-Aldrich Chemical Co. (Oakville, ON, Canada). All chemicals were reagent grade and used without further modification.

Preparation and Stability of WSC-FA-ePC Blend

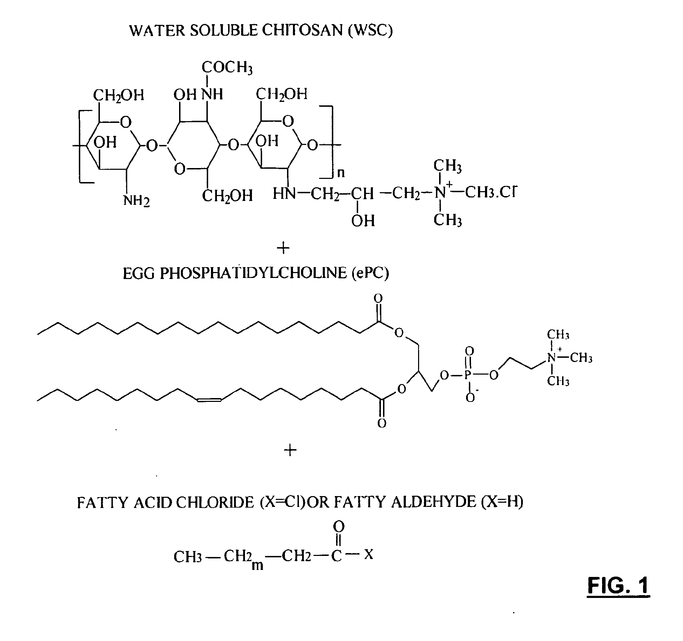

The WSC derivative composed of GTMAC and chitosan in a ratio of 3:1 mol/mol was synthesized using a previously established method. (Cho, J., et al., Biomacromolecules, 2006. 7(10): p. 2845-2855; Seong, H.S., H.S. Whang, and S.W. Ko, Journal of Applied Polymer Science, 2000. 76(14): p. 2009-2015.)
Following the purification procedure, the WSC derivative was weighed and then dissolved in distilled water. The lipid ePC was solubilized with FA varying in acyl chain length and added to the WSC solutions in specific weight ratios. Lastly, the WSC-FA-ePC blend was vortexed for two minutes and stored at room temperature. For preparation of the drug loaded blends, $5\mu$Ci of the $C^{14}$-PTX in ethyl acetate was added to 10 mg of PTX and dried under nitrogen to form a thin film of drug. A FA-lipid solution containing C12, C14 or C16 FA and ePC was used to resuspend the PTX film prior to mixing with WSC to achieve a WSC:FA:ePC:PTX (1:4:1:0.25 w/w/w/w) blend.
The stability of the samples was measured as a function of turbidity, which is the amount of light that a sample scatters. Approximately 300 $\mu$L of the WSC-FA-ePC blend was injected into a vial containing 0.01 M PBS and 0.2% lysozyme since chitosan degrades in the presence of lysozyme *in vivo*. At specific time points, an aliquot of the solution was added to a cuvette and analyzed using UV-vis spectroscopy (Cary™ 50 UV-vis spectrophotometer, Varian Inc., Palo Alto, CA) at 700 nm (no absorption occurred for the blend at this wavelength). The aliquot was then transferred back into the vial containing the blend for subsequent analysis. The stability of the WSC-FA-ePC blends was also visually inspected during preparation and following injection into 0.01 M PBS over a 72 hour period at 37°C.
In order to dissolve chitosan in the absence of an acidic solution, chitosan was modified to a WSC derivative using GTMAC. Previously, FTIR analysis revealed that the lower the degree of substitution of GTMAC onto chitosan chains, the greater the interaction between ePC and the $NH_2$ groups of WSC. From the series of WSC synthesized, the 56% DS WSC was the most suitable polymer for preparing the blend formulations. Different concentrations and chain lengths of FA were mixed with WSC and ePC to increase the overall hydrophobicity and subsequently the stability of the formulation in aqueous environments. In addition, WSC acts as a surfactant for FA as it prevents phase separation that occurs when FA is in contact with distilled water. The chemical structures of each of the components within the blend, namely WSC, FA and ePC are shown in Fig. 1.
Upon mixing all the materials, the stability of the WSC-FA-ePC blend was found to be dependent on the acyl chain length of the FA employed and the ratio of the three components. Specifically, the WSC-FA-ePC blend containing the C12 FA was unstable in buffer solution when the amount of WSC was below 1:4:1 and above 1.5:4:1 (w/w/w) blend ratios. At low concentrations of WSC, there were insufficient reactive groups to stabilize the blend; whereas, at high concentrations of WSC, the viscosity of the blend was increased and the miscibility of the components was reduced.

The stability of the blend was also found to be dependent on the amount of ePC, as a mixture of WSC and FA dissolved in the presence of buffer. A minimal amount of ePC (e.g. 1:4:0.5) was required to form a stable blend that remained intact following incubation in buffer for 72 hours. In addition, the amount of FA was crucial to the stability of the blend, since a formulation containing only WSC-ePC would dissolve rapidly in an aqueous environment as discussed above. However, blends that contained FA below the 1:4:1 (WSC:FA:ePC, w/w/w) ratio were unstable following the incubation period.

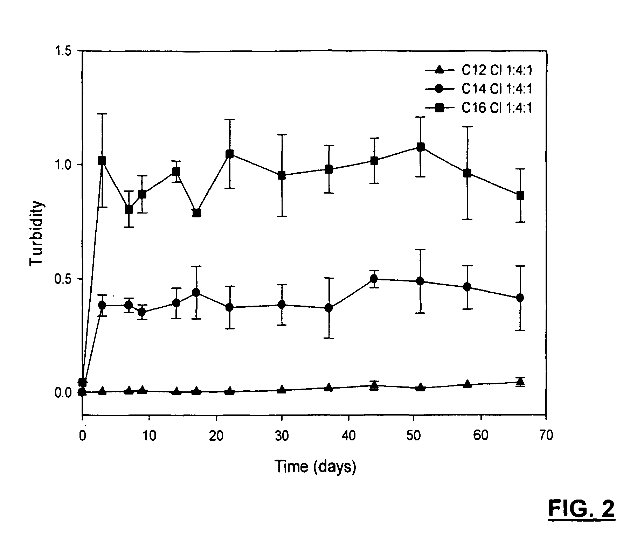

The effect of acyl chain lengths of FA ranging from C6-C16 was investigated for the 1:4:1 (w/w/w) WSC-FA-ePC blend. During preparation, the blend containing either the C6 FA or C8 FA easily solubilized ePC, yet the solution phase separated in the presence of WSC. The blends containing FA of C10-C16 in chain length formed stable formulations, but differed in terms of their mechanical properties. Specifically, the blends containing C10-C12 FA were semi-solid, whereas the C14-C16 FA blends solidified upon mixing. However, following 24 hours at room temperature, the blends containing the C14-C16 FA formed a viscous solution with reduced mechanical properties. Therefore, the C12 FA was considered the optimal chain length for preparation of a stable semi-solid injectable blend. This result was confirmed by analyzing the turbidity of the WSC-FA-ePC blends as a function of chain length in physiological solution (Fig. 2). From the results, the C10 FA blend disintegrated upon injection into buffer (data not shown). However, the C12-C16 blend formulations were stable as the turbidity was less than 0.1 after 1 hour. The C12 FA blend was the most stable formulation as the turbidity did not exceed 0.05 for 2 months. The C14 FA blend also remained stable as a low turbidity (< 0.5) was observed for the duration of the experiment. However, the C16 FA blend was unstable as the turbidity increased to ca. 1 after 3 days of incubation.

Thermal Analysis of the WSC-FA-ePC Blend

A DSC™ Q100 (TA Instruments, New Castle, DE) was used to determine the thermal transitions of the WSC-FA-ePC blends. Samples of 5-7 mg were placed in hermetic pans and their transition temperatures were analyzed between -40°C to 80°C at a temperature ramp speed of 5°C per minute under nitrogen purge. TA universal analysis software was used to analyze the second heating cycle of all samples.

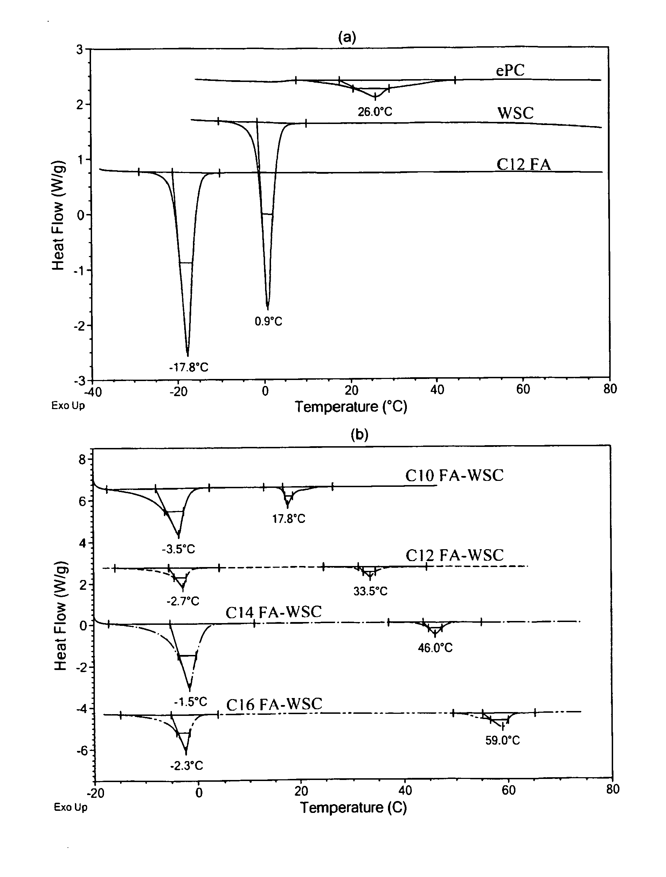

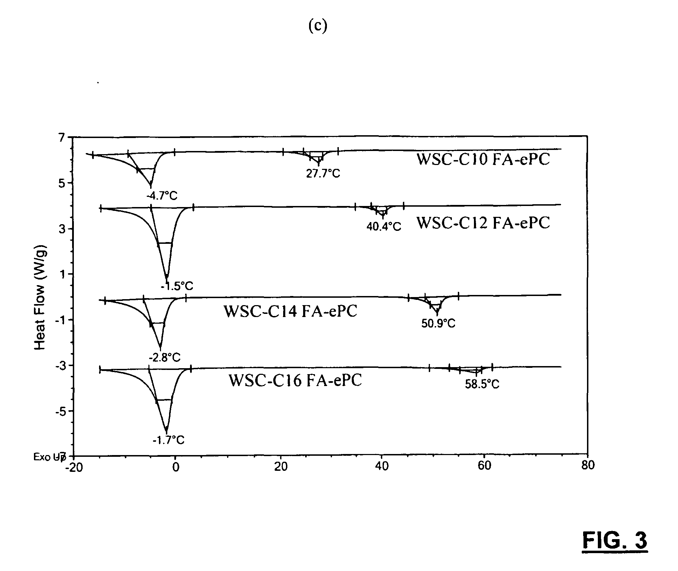

The thermal behavior of the blends containing WSC, FA and/or ePC was evaluated in order to examine the miscibility of the three components and to determine the stability of the blends at body temperature (Fig. 3). For WSC alone, only an endothermic peak representing the water content was present at approximately 0°C (Fig. 3a). This peak appeared for each sample containing WSC, but did not interfere with the melting transitions of ePC and FA. At approximately 26°C, a single broad melting transition ($T_m$) was observed for ePC alone due to the heterogeneity of this lipid (i.e. contains hydrocarbon chains varying in length and degrees of saturation) as shown in Fig. 3a. In order to prepare the WSC-ePC blend, the lipid was dissolved in ethanol and then mixed with WSC. For the WSC-ePC blend (4:1, w/w), no thermal transitions were observed for ePC (data not shown).

However, fatty acids generally have a sharp transition that can vary from 2°C to 64°C depending on the acyl chain length (see Table 1). For C12 FA alone, a melting transition at -18°C was observed due to the chloride ion (Fig. 3a). The addition of WSC to FA resulted in the dissociation of the chloride ion, which in turn increased the melting transitions for the C10 to C16 FA to range from 18°C to 59°C, respectively (Fig. 3b). As discussed above, the WSC-FA blend formed a gel that was unstable in buffer, thus ePC was added to enhance the stability of the formulation. The thermal behavior for WSC-FA-ePC blends varying with FA acyl chain length is shown in Fig. 3c. The FA and ePC components were considered to be miscible as only a single peak was observed for these materials in the temperature range investigated. Similar to the WSC-FA blends, the peak position was found to increase in temperature from 28 to 58°C with increasing FA acyl chain length. However, the peak positions for WSC-FA-ePC blends were more comparable to the values obtained in the literature for C10-C14 fatty acids (Table 2). No significant changes in melting transition were observed for the C16 blends (i.e. WSC-FA and WSC-FA-ePC). Thus, WSC-FA-ePC blends containing FA greater than an acyl chain length of C10 had thermal transitions above body temperature. However, ePC may have less interaction with longer acyl chain length FA, which may explain the instability observed for the WSC-FA-ePC blend containing the C16 FA (Fig. 2).

Table 2. The Physical properties of various fatty acid chlorides.

| Fatty acid | Molecular Formula | $L_C$ | $T_m$ (°C) | MW (g/mol) | Density (g/cm³) |
|---|---|---|---|---|---|
| Decanoyl chloride | $CH_3(CH_2)_8COCl$ | 10 | 230-232 | 191 | 0.93 |
| Lauroyl chloride | $CH_3(CH_2)_{10}COCl$ | 12 | 134-137 | 219 | 0.92 |
| Myristoyl chloride | $CH_3(CH_2)_{12}COCl$ | 14 | 250 | 247 | 0.91 |
| Palmitoyl chloride | $CH_3(CH_2)_{14}COCl$ | 16 | NA | 275 | 0.91 |

(continued)

| Fatty acid | Molecular Formula | $L_C$ | $T_m$ (°C) | MW (g/mol) | Density (g/cm$^3$) |
|---|---|---|---|---|---|
| Laurinaldehyde | $CH_3(CH_2)_{10}CHO$ | 12 | 185 | 184 | 0.83 |

FTIR Analysis of the WSC-FA-ePC Blend

The FTIR spectra of the WSC-FA-ePC blends and their individual components were obtained using a universal ATR Spectrum-one spectrophotometer (Perkin-Elmer, Wellesley, MA). A background spectrum of air was subtracted from the sample spectra using Perkin-Elmer's Spectrum software. All spectra were an average of 16 scans at a resolution of 2 cm$^{-1}$, repeated in triplicate.

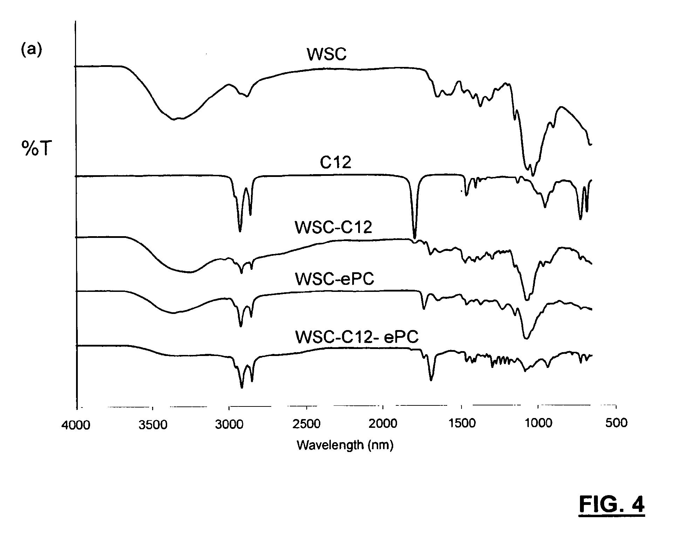

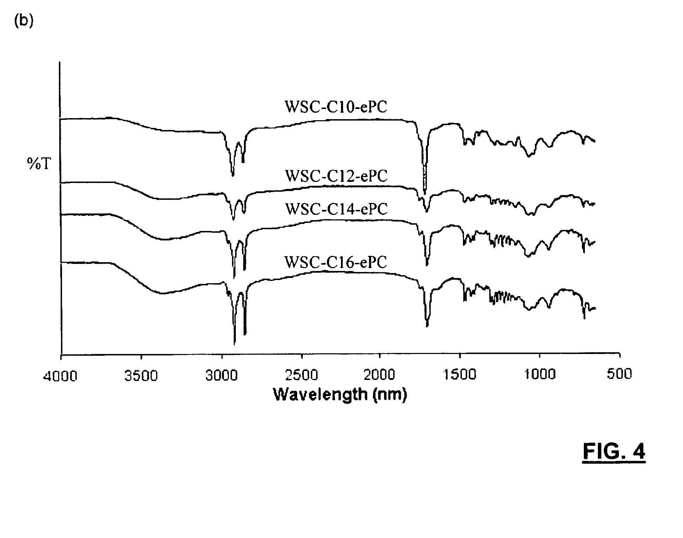

In order to determine the interactions that stabilize the WSC-FA-ePC blends, FTIR spectra of the blends and the individual components that make up the blends were analyzed (Figs. 4a and 4b). For WSC (56% DS), the conjugation of GTMAC to chitosan was confirmed by the appearance of the peak at 1475 cm$^{-1}$, which represents the methyl band of GTMAC and the peak at 1564 cm$^{-1}$ corresponds to the primary amine group of chitosan. Previously, FTIR analysis established that an interaction exists between the WSC (56% DS) and ePC as the peak corresponding to the NH$_2$ groups of WSC shifted from 1564 to 1575 cm$^{-1}$ with the addition of ePC. The spectra of the C12 FA contained a sharp peak at 1800 cm$^{-1}$ which represents the acid chloride groups (COCl), and peaks at 1291 cm$^{-1}$ and 717 cm$^{-1}$ which correspond to C-O and C-Cl bonds, respectively (Fig. 4a).

For the WSC-C12 FA blend, the peak at 1800 cm$^{-1}$ contributed from the FA was significantly reduced in area and a new peak appeared at approximately 1700 cm$^{-1}$ (Fig. 4a). This new peak, which may represent a carboxylic acid group that formed during the dissociation of the chloride ion, became even more prominent with the addition of ePC to the WSC-FA blend. The displacement of the chloride ion is further evidenced by the significant reduction in area of the C-Cl peak at 717 cm$^{-1}$. The NH$_2$ groups of WSC were very difficult to detect for the WSC-FA-ePC blend. The area of the peaks representing the OH groups at 3300 cm$^{-1}$ was smallest for the WSC-FA-ePC in comparison to the WSC-FA and WSC-ePC blends. In addition, a large number of small peaks were observed between 1200 to 1400 cm$^{-1}$ that may be contributed from the C-O bonds from each of the FA, as well as the choline headgroup from the lipid. However, it is postulated that the NH$_2$ groups of WSC, the CO and CH$_2$ groups of FA and the phosphatidylcholine headgroup of ePC are all involved in stabilizing the blend formulation.

The interactions within the blend were also investigated by FTIR as a function of FA chain length (Fig. 4b). For the spectra of C10 to C16 FA, the peak positions for each of the FA spectra were nearly identical (data not shown). Similarly, no obvious differences were shown in the spectra for WSC-FA blends varying in FA chain length (the spectra for WSC-C12 FA is shown in Fig. 4a). However, for the WSC-FA-ePC blends, the number of peaks between 1200 to 1400 cm$^{-1}$ increased with increasing FA chain length (Fig. 4b). Furthermore, the peak at 1700 cm$^{-1}$ for the C12, C14 and C16 FA blends was found to shift to 1708 cm$^{-1}$ for the C10 blend. In addition, the peak area at 1700 cm$^{-1}$ was significantly decreased for only the C12 and C14 WSC-FA-ePC blends. Thus, the stability found for the WSC-FA-ePC blend containing C12 and C14 FA may be related to the interactions present at the carboxylic acid groups of FA.

Optical and Fluorescence Microscopy of the WSC-FA-ePC Blend

Images of the WSC-FA, WSC-ePC and WSC-FA-ePC blends were captured using a Zeiss™ Axiovert 135 TV light microscope. The chitosan and lipid regions were identified in the WSC-FA-ePC blends containing C10, C12 and C16 FA by an inverted two photon confocal laser scanning fluorescence microscope (Zeiss™ LSM 510 META NLO, Germany). Briefly, 1 mol % of the fluorescent phospholipid NBD-DPPE ($\lambda_{ex}$ = 460 nm, $\lambda_{em}$ = 534 nm) was dissolved in ethanol and dried to a film using nitrogen. Pure ePC was dissolved in FA and mixed with the fluorescent lipid film. Fluorometric measurements revealed that the WSC solution has a strong autofluorescence signal ($\lambda_{ex}$ = 400 nm, $\lambda_{em}$ = 800 nm). Thus, the fatty acid-lipid solution was blended with WSC and cast onto a glass slide. Cover slips were placed on the solution to prevent optical reflectance and the formulation was dried overnight in a dark environment.

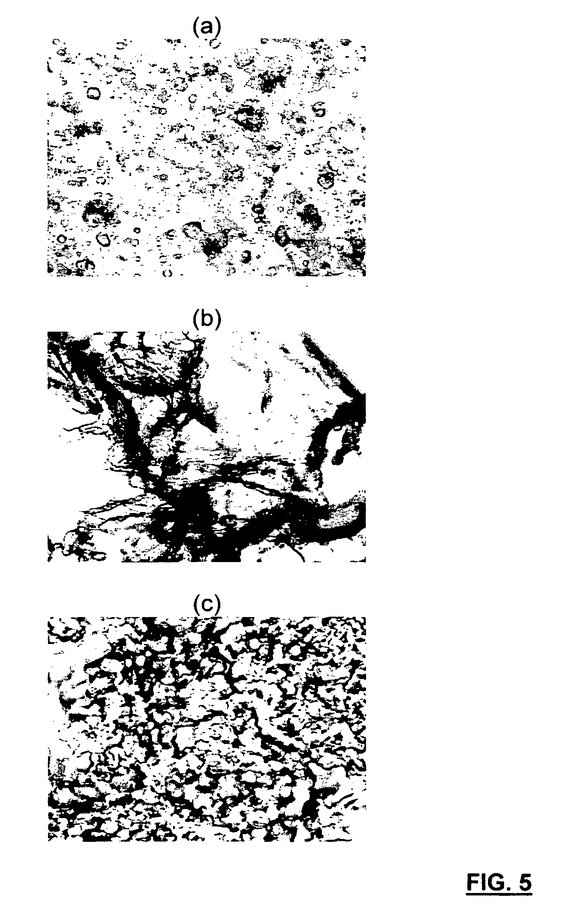

The morphology of WSC-FA-ePC blends at various weight ratios was visualized by light microscopy as shown in Fig. 5. From the images, blends of WSC and ePC form a partially miscible blend with domains of lipid throughout the chitosan matrix (Fig. 5a). In a previous report, it was determined that the size of the lipid domains within a chitosan matrix increased with increasing amounts of ePC. As shown in Fig. 5b, the WSC-FA C12 blend had no apparent structural order and microphase separation. However, when the WSC, FA and ePC were combined in a 1:4:1 (WSC-FA-ePC, w/w/w) ratio, a tighter organization of the components was observed (Fig. 5c). Thus, from the images, all three components (WSC, FA and ePC) were necessary in the formation and stabilization of the blend.

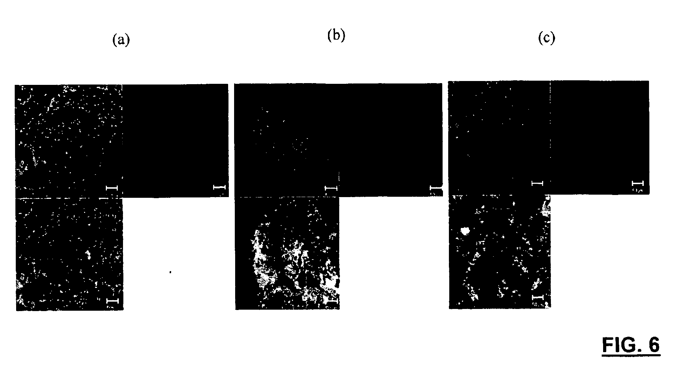

Laser scanning fluorescence microscopy was used to identify the lipid and WSC regions as well as determine the effect of increasing the FA acyl chain length within the WSC-FA-ePC blend (Fig. 6). From the images, the red regions represent the WSC component of the film and the bright green fluorescent regions correspond to the lipid and/or FA. The yellow regions represent areas of co-localization or interaction between the components of the blend, while the black regions may correspond to the unlabelled FA or the uneven surface of the blend. For the WSC-FA-ePC (1:4:1, w/w/w) blend containing C10 FA, the domains for both WSC and the FA-ePC appear small and scattered throughout the blend with minimal areas of interaction (Fig. 6a). This morphology may provide an indication of the instability that was observed for this blend by turbidity measurements. However, the blend containing the C12 FA has larger domains for both WSC and FA-ePC, as well as a higher degree of interaction between the components as evidenced by the large yellow regions (Fig. 6b). The larger domains may be due to the increase in hydrophobicity of the FA employed within the blend. This morphology may explain the increase in rheological properties and stability for the blends containing longer FA chains. However, a larger degree of phase separation was apparent for the WSC-FA-ePC blend containing the C16 FA as the lipid-FA domains appear disjointed with a distinct spatial arrangement (Fig. 6C). Therefore, the acyl chain length of the FA employed within the WSC-FA-ePC blend has a significant effect on molecular arrangement of the molecules.

Rheological Measurements of the WSC-FA-ePC Blend

The rheological properties of WSC-FA-ePC blends were characterized by a stress-controlled rheometer with a 2 cm cone and 4° angle plate geometry (AR-2000, TA Instruments™). The rheometer was calibrated and rotational mapping was performed according to instrument specifications. The viscosity was measured using a continuous ramping flow mode while increasing the shear stress from 1 to 500 Pa. The blend formulations were stored for 24 hours prior to mechanical testing. A 200 $\mu$L injection of each sample was placed on the rheometer plate for mechanical testing.

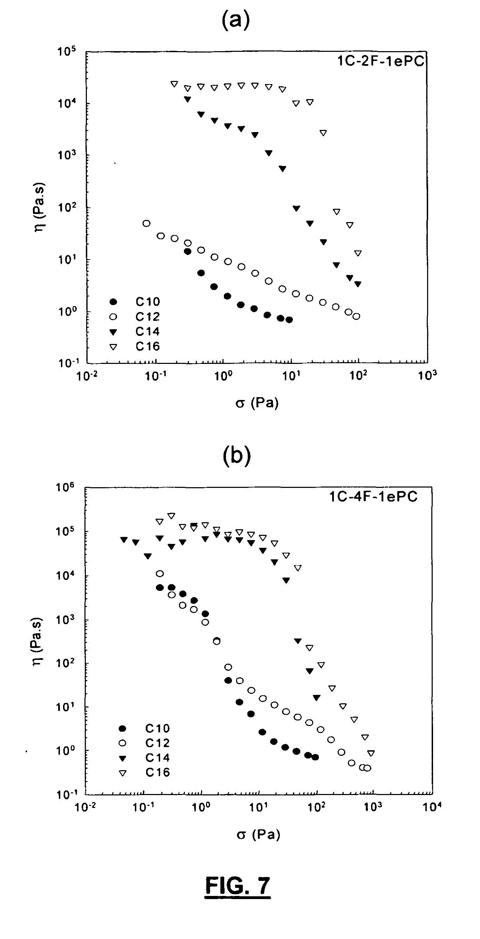

The rheological properties of injectable systems are important for drug formulations as a blend with low viscosity may not exhibit a sustained drug release profile. However, if the blend is too viscous, difficulties will be encountered when injecting the formulation. n order to determine the optimal rheological properties of the injectable system, the viscosities of the WSC-FA-ePC blends (1:2:1 and 1:4:1) were measured as a function of the FA chain length using steady shear tests (Fig. 7). From the results, as the hydrocarbon chain length of the FA increased within the blends, the viscosity and yield stress values increased. For the 1:2:1 WSC-FA-ePC blends, the blend containing the C10 FA had the lowest viscosity and yield stress values, as well as a slight increase in viscosity ($\sim 1 \times 10^2$ Pa) was found for the blend containing the C12 FA (Fig. 7a). For the blends containing the C14 and C16 FA, a significant increase in viscosity to approximately $1 \times 10^4$ Pa was measured. When the amount of FA was increased within the WSC-FA-ePC blend to 1:4:1, a similar trend was observed at higher viscosity and yield stress values (Fig. 7b). Specifically, the blends containing the C14 and C16 FA had a viscosity of approximately $1 \times 10^5$ Pa, whereas the C12 and C10 FA blends were approximately $1 \times 10^4$ Pa. It is known that most injectable systems use a needle size no larger than 22 gauge, otherwise special equipment such as hydraulic syringes are employed. From these results, only the WSC-FA-ePC (1:4:1) blends containing C10 and C12 FA were injectable via a 22 gauge needle; the C14 and C16 FA blends were too viscous. Thus, from the stability and rheological analyses, the optimal formulation ratio to produce a viscous injectable blend was found to be 1:4:1 (w/w/w) WSC-FA-ePC containing the C12 FA.

Release of $^{14}$C-PTX from the WSC-FA-ePC Blend

Approximately 300 $\mu$L of the WSC-FA-ePC blend, which contained a mixture of $^{14}$C-PTX (0.14 $\mu$Ci) and cold PTX (10 mg), was injected into 5 mL of 0.01 M PBS with 0.2 % lysozyme and incubated at 37°C. At specific time points, 2 mL of solution was removed from the vial and subsequently replaced with 2 mL of fresh PBS/lysozyme solution. A 4 mL aliquot of Ready Safe™ liquid scintillation cocktail (Beckman Coulter Inc., Fullerton, CA) was added to each sampled aliquot, vortexed and analyzed by scintillation counting (Beckman™ LS 5000 TD, Beckman Instruments Inc., Fullerton, CA).

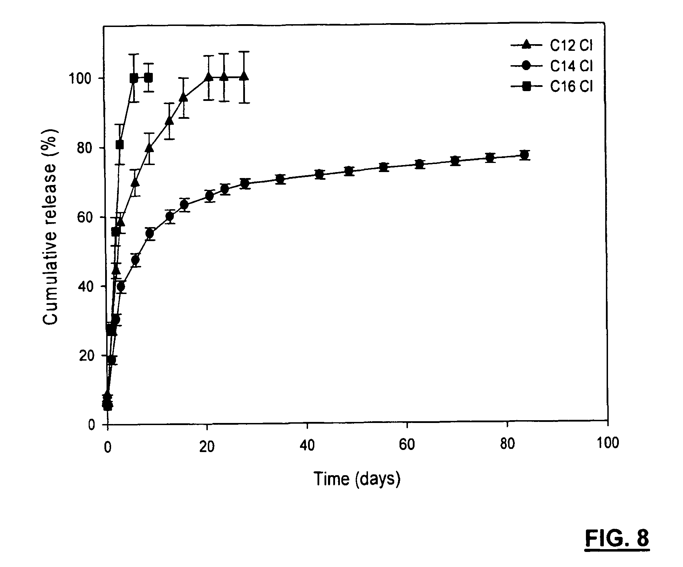

The release of $^{14}$C-PTX from the WSC-FA-ePC (1:4:1, w/w/w) blends as a function of FA acyl chain length in 0.01 PBS containing lysozyme is shown in Fig. 8. During the first 24 hours of analysis, an initial release of PTX ranging from 19 to 28% was observed for the WSC-FA-ePC blends prepared from C12 to C16 FA. After 7 days, the release from the C12 FA blend was approximately 70% compared to ca. 50% for the C14 FA blend and 100% for the C16 FA blend. The complete release (i.e. 100%) of PTX from the C12 FA blend was observed at 21 days. This may be an optimal drug release period as current treatment regimens for patients with ovarian cancer, non-small cell lung cancer and breast cancer are given Taxol (paclitaxel in Cremophor® EL (polyoxyethylated castor oil) and dehydrated alcohol) intravenously, as part of their chemotherapy every three weeks. In the case of the C14 FA blend, the release reached ca. 70% after 35 days, and continued at a sustained release rate of 0.2 % per day for 3 months. Similar trends have been observed in other studies.

The kinetics of PTX release from WSC-FA-ePC injectable blends was characterized using three different models:

$$\text{Higuchi equation: } \frac{M(t)}{M(0)} = K_H t^{0.5} \tag{1}$$

$$\text{Ritger-Peppas equation: } \frac{M(t)}{M(0)} = K_{R\_P} t^{n} \tag{2}$$

$$\text{Peppas-Sahlin equation: } \frac{M(t)}{M(0)} = K_{P\_S,1} t^{m} + K_{P\_S,2} t^{2m} \tag{3}$$

where $K_H$, $K_{R\_P}$, $K_{P\_S,1}$ and $K_{P\_S,2}$ are empirical constants related to the release rate, M(t)/M(0) is the fraction of drug release and t is the time of release. In equation 2, $n$ is the diffusional exponent which indicates the main mechanism of drug release. In equation 3, the first term represents a Fickian diffusion contribution and the second term is the effect of Case II-transport on drug release.

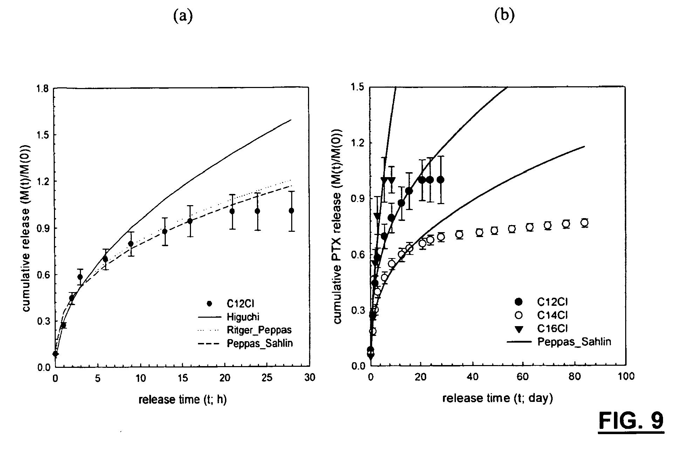

The release of PTX from the C12 FA blend was fitted to the various kinetic models (Higuchi, Ritger-Peppas and Peppas-Sahlin) as shown in Fig. 9. The Higuchi model was applied only up to 50% of drug release, but had a good correlation ($r^2 = 0.95$). The release profile of PTX was also a good fit for the Peppas-Sahlin and Ritger-Peppas models ($r^2 = 0.98$ - 0.99) except in the regions in which very little release was observed.

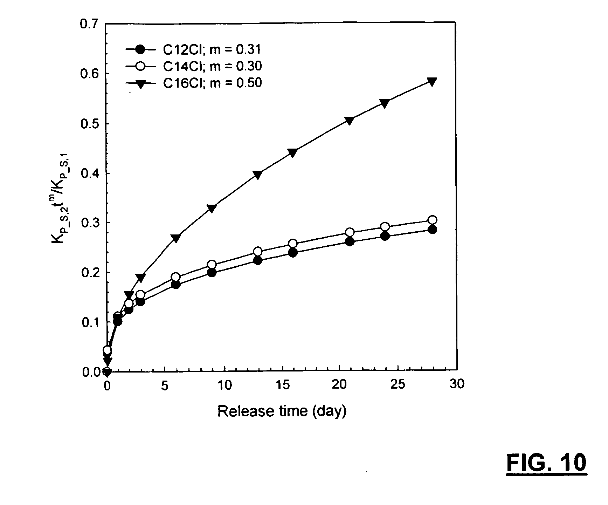

The values of each of the parameters from the three drug release kinetic equations are shown in Table 3. Regardless of the release model, the K value, which is related to the drug release rate, was highest for C16 and lowest for C14. From the Peppas-Sahlin equation, the n values calculated were less than 0.43 for C12 FA (i.e. $n = 0.37$) and C14 FA (i.e. $n = 0.36$) blends, which is indicative of a release that has occurred by Fickian diffusion through a polymeric matrix. In the case of the C16 FA blend, $n$ was 0.58, which is corresponds to a non-Fickian release behavior. Drug release is contributed by two parameters, namely Fickian diffusion and Case II-transport. The specific contribution can be determined by plotting the $K_{P\_S,2} t^{m} / K_{P\_S,1}$ value as a function of time as shown in Fig. 10. Due to the large deviation in the fitting, release data greater than 28 days was not included in the plot. Fickian diffusion is dominant when $K_{P\_S,2} t^{m} / K_{P\_S,1} < 1$, but Case II-transport is the main contributor to release when $K_{P\_S,2} t^{m} / K_{P\_S,1} > 1$.[36] From Fig. 10, $K_{P\_S,2} t^{m} / K_{P\_S,1}$ remained less than 1, which is indicative that Fickian diffusion seems to be the main contributor of drug release for the WSC-FA-ePC blend systems. However, for C16 FA blend, the ratio $K_{P\_S,2} t^{m} / K_{P\_S,1}$ increased rapidly in comparison to the C12 FA and C14 FA blends. Thus, it implies that the Case II-transport contribution could have influenced the drug release of PTX from the C16 FA blend.

Table 3. Values of release parameters determined from various kinetic models for 1:4:1 (w/w/w) WSC-FA-ePC blends varying in acyl chain length

| Sample | Higuchi | Ritger-Peppas | | Peppas-Sahlin | | |
|---|---|---|---|---|---|---|
| | $K_H$ | $K_{R\_P}$ | $n$ | $K_{P\_S,1}$ | $K_{P\_S,2}$ | $m$ |
| C12 FA | 0.30 | 0.35 | 0.37 | $3.23 \times 10^{-1}$ | $3.24 \times 10^{-2}$ | 0.31 |
| C14 FA | 0.21 | 0.24 | 0.36 | $2.20 \times 10^{-1}$ | $2.44 \times 10^{-2}$ | 0.30 |
| C16 FA | 0.41 | 0.37 | 0.59 | $3.35 \times 10^{-1}$ | $3.69 \times 10^{-2}$ | 0.50 |

The higher release rate (or large $K$ value) observed for the C16 FA blend may be attributed to the greater immiscibility that was observed from the confocal images and turbidity results (Figs. 2 and 6). When the chain length of the FA was increased, the overall hydrophobicity of the blend was also increased. The longer fatty acids aggregate into the hydrophilic environment (i.e. aqueous WSC solution), resulting in a phase separated blend. Phase separation causes a reduction in volume (or size) of the blend, which in turn may lead to an increased surface area and a reduced diffusion length for the drug.

The rheological properties of the blends may also explain the higher release rate of paclitaxel from the C16 blend. The rheological properties were increased when a longer FA was used to formulate the blend system (i.e. $\eta \sim 100$ Pas and $\eta \sim 10^5$ for C14 and C16, respectively). In general, the viscosity increases when there are more interactions between macromolecules in a polymeric system such as entanglement, physical interaction (i.e. Van der Waals, hydrophobic and hydrogen bonding interactions) and cross-linking. These interactions between the macromolecules can be used to trap the drug in a polymer matrix. Thus, the more interactions present in a polymer system, the rate of drug release will be slower. Overall, the C12 FA had the lowest viscosity among the three blends, indicating the lowest connectivity, resulting in an accelerated PTX release.

It is known that the solubility of the drug in a formulation can also strongly influence the drug release performance. The drug release from C12 FA was faster than C14 FA, since PTX was more soluble in the shorter FA acyl chains. The slow drug release exhibited by C14 FA blend may also be due to high viscosity (high connectivity) and minimal phase separation between the hydrophilic and hydrophobic components in the matrix. Depending on the specific treatment that is required, the drug release can be controlled by modifying either the type of FA used or the specific concentrations of each of the individual components in the blend.

EXAMPLE 2

Composition with Lauroyl Chloride

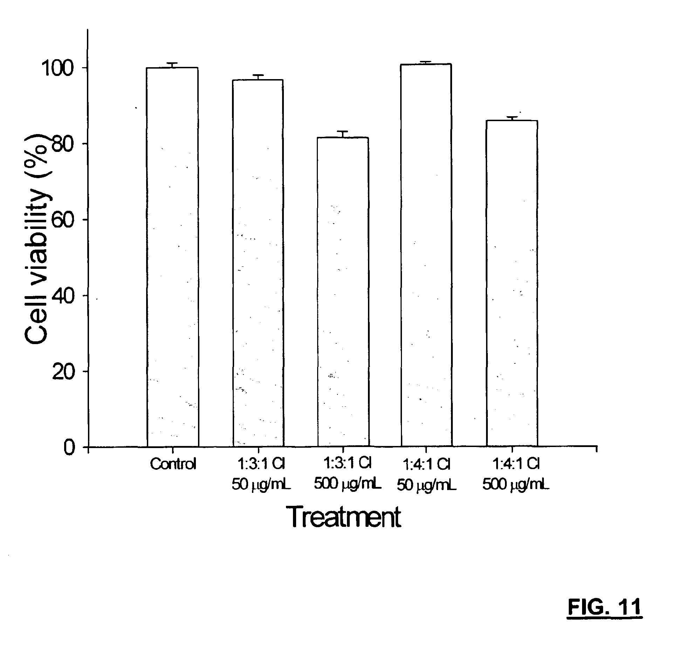

An injectable polymer-lipid blend including lauroyl chloride was prepared to assess cell viability. The composition included a low molar weight WSC, C12 Cl and ePC in ratios of 1:3:1 and 1:4:1. The ePC was dissolved with lauroyl chloride (C12 Cl) and vortexed to dissolve. Water soluble chitosan (WSC) was then added, and the blend was further vortexed and then transferred to a 1 cc syringe. The composition in the syringe was then sterilized with UV sterilizer for 24 hours. The *in vitro* biocompatibility of L929 cells in the presence of the blends (1:3:1 and 1:4:1) were assessed after 24 hours of incubation, as shown in Fig. 11. Error bars are expressed as standard error (n=12).

EXAMPLE 3

Blend with Laurinaldehyde

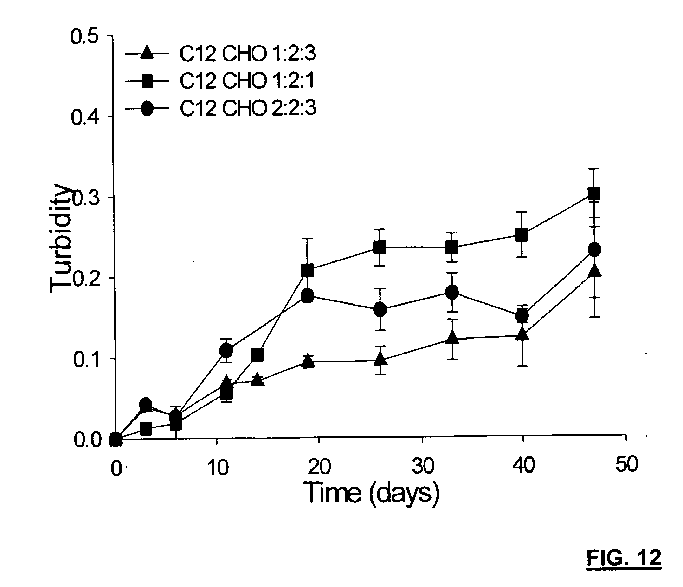

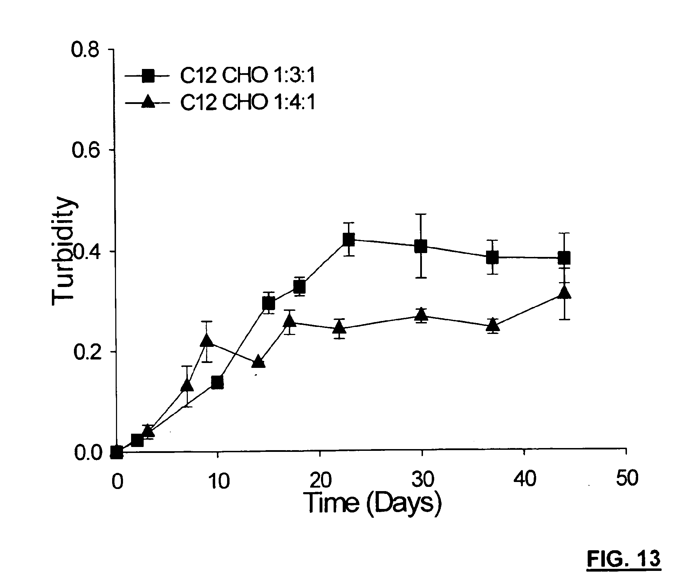

A composition was prepared including WSC (low MW), laurinaldehyde and ePC in ratios of 1:2:1, 1:2:3, 2:2:3, 1:3:1 and 1:4:1. The compositions were prepared by dissolving ePC with the laurinaldehyde and vortexed, followed by the addition of WSC and further vortexed to blend components, and then transferred to a 1 cc syringe. As an indication of stability, turbidity of the blend formulations, incubated in 0.01 M phosphate buffer solution (pH = 7.4) containing 2 mg/mL lysozyme at 37°C, is shown in Fig. 12 and Fig. 13.

Further compositions were prepared comprising WSC (low and high MW), laurinaldehyde and ePC in ratios of 1:3:1 and 1:4:1 along with 10 mg of paclitaxel. The paclitaxel was added in ethanol and the solvent was fully evaporated. The components were vortexed in stages before transferred to a 1 cc syringe. Partition coefficients (K$_v$) of paclitaxel according to the formula:

$$K_V = \frac{PTX\ conc.(blend)}{PTX\ conc.(solution)}$$

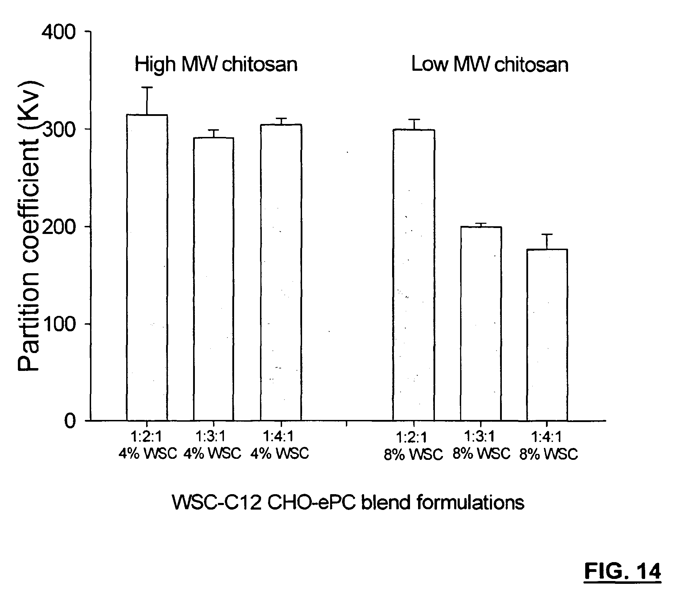

in the various blend formulations incubated in 15 mL of 0.01 M phosphate buffer solution (pH = 7.4) at 37°C for 7 days were calculated as shown in Fig. 14.

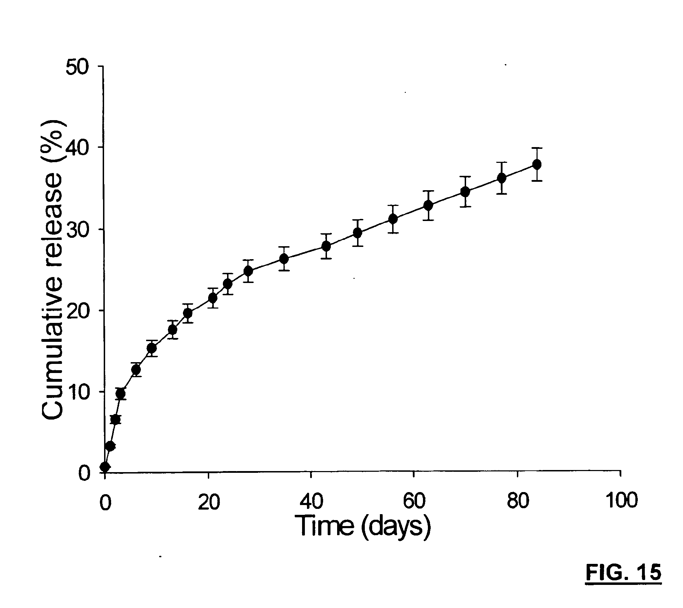

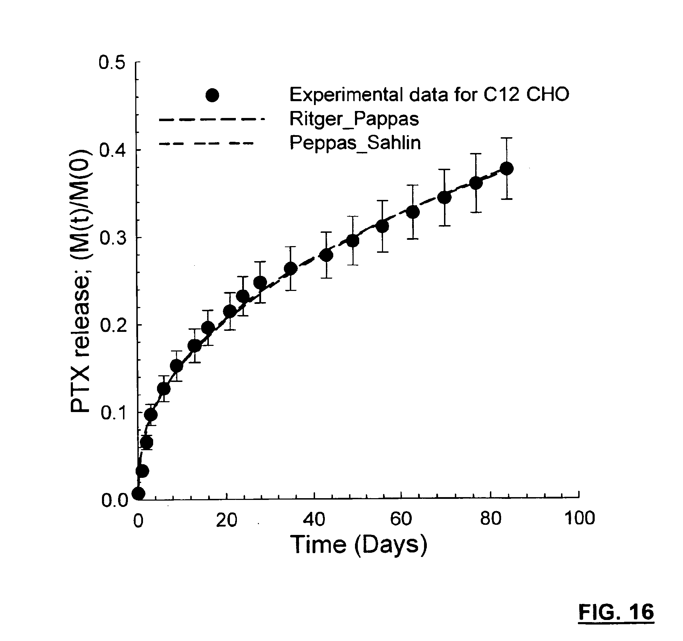

Compositions also were prepared comprising WSC (high MW), laurinaldehyde and ePC in a ratio of 1:4:1, along with 10 mg of paclitaxel and 14C-paclitaxel. The 14C-paclitaxel (14C-PTX) and paclitaxel were added in ethanol and the solvent fully evaporated. Laurinaldehyde was used to dissolve ePC. The components were vortexed in stages and then transferred to a 1 cc syringe. The percent of cumulative release of 14C-PTX-labeled paclitaxel from various blend formulations incubated in 0.01 M phosphate buffer solution (pH = 7.4) containing 2 mg/mL lysozyme at 37°C are shown in Fig. 15. Ritger-Peppas and Peppas-Sahlin release models fit to the cumulative release of 14C-PTX/PTX from the WSC/C12 CHO/ePC blend formulations are shown in Fig. 16.

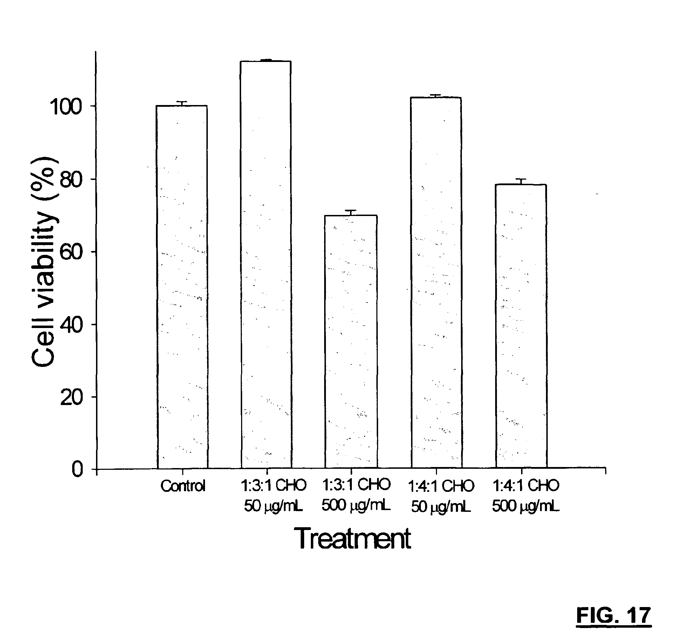

Further compositions were prepared comprising WSC (low MW), laurinaldehyde and ePC in ratios of 1:3:1 and 1:4:1. The ePC and laurinaldehyde were vortexed, and then WSC was added. The blend was further vortexed and then transferred to a 1 cc syringe, and sterilized with UV sterilizer for 24 hours. The *in vitro* biocompatibility of L929 cells in the presence of the blends (1:3:1 and 1:4:1) after 24 hours of incubation is shown in Fig. 17.

Yet further compositions were prepared comprising WSC (low MW), laurinaldehyde and ePC in a ratio of 1:4:1. The ePC and laurinaldehyde were vortexed, and then WSC was added. The blend was further vortexed and then transferred to a 1 cc syringe, and sterilized with UV sterilizer for 24 hours. The *in vivo* biocompatibility of blends (1:4:1) after injection into the intraperitoneal cavity of female CD-1 mice (n=3) for up to 4 weeks was established as indicated in Table 4. Alanine aminotransferase (ALT) activity in the plasma was determined after 4 weeks (n=3). Acceptable ALT levels are considered to be 10-35 U/L.

Table 4. The physical properties of various fatty acids.

| Treatment | Average Absorbance (A) | SE (+/-) | U/L |
|---|---|---|---|
| A. C12-CHO + saline | 0.979 | 0.008 | 21.8 |
| B. C12-CHO + saline | 0.974 | 0.010 | 28.8 |
| C. C12-CHO + saline | 1.041 | 0.007 | 20.1 |
| saline only | 0.902 | 0.011 | 31.3 |

EXAMPLE 4

*In Vitro* Release Profile of Docetaxel (DTX) and *In Vivo* Biocompatibility of Drug-free and Drug-loaded Blends in CD-1 Mice

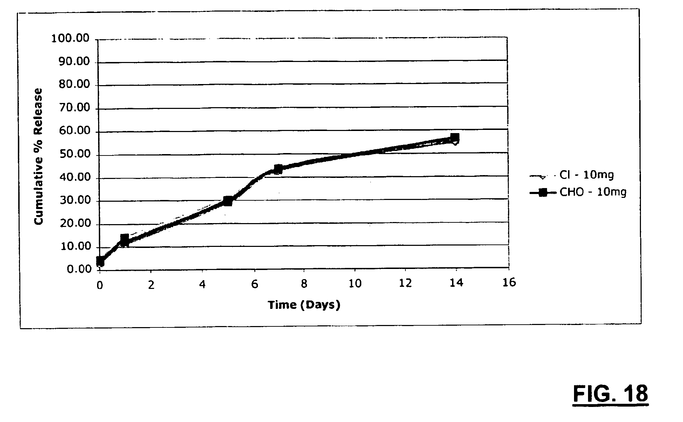

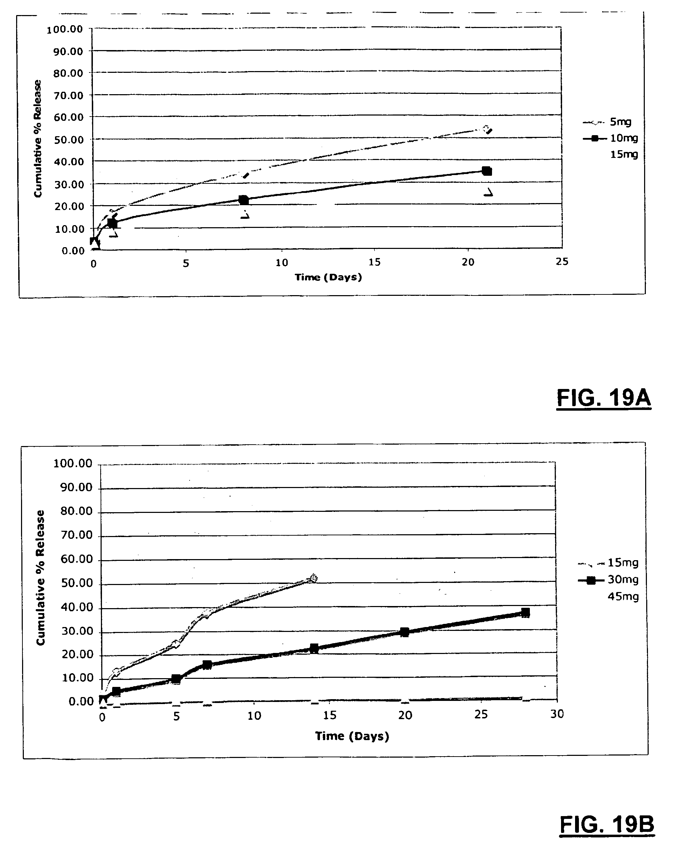

Compositions were prepared comprising WSC (high MW), C12Cl or C12-CHO and ePC in a ratio of 1:4:1 along with DTX in the following amounts: 5-45 mg. The drug release profile of 10 mg of $^3$H-DTX from two different formulations is provided at Fig. 18. *In vitro* drug release profiles for further formulations comprising C12-CHO and DTX illustrate the concentration effect of different amounts of loaded H-DTX in the CHO formulation, as shown in Figs. 19A and 19B.

*In vitro* release of $^3$H-DTX from the blend formulation (1:8 w/w, drug/formulation) was determined by incubating the formulation in 0.01 M phosphate-buffer solution and 2 mg/mL lysozyme. At specific time points, an aliquot was analyzed by liquid scintillation counting. Female CD-1 mice were injected in the intraperitoneal cavity with either a drug-free or a DTX-loaded (8 mg/kg) blend formulation. Following weekly sacrifices for 7 weeks, mice were visually inspected for signs of intestinal inflammation or capsid formation. Steady state DTX plasma concentrations were determined by HPLC analysis. Liver toxicity was assessed by measuring alanine aminotransferase (ALT) levels.

*In vitro* release of $^3$H-DTX was shown to be 4.6% per day (n=3) over 3 weeks. *In vivo* biocompatibility of drug-free and drug-loaded blends was confirmed in the mice. They appeared healthy without any significant weight loss. Post-mortem examination did not show local peritoneal inflammation. Minimal to no capsid formation was observed. Sustained DTX plasma levels of 0.033 $\pm$ 0.01 ug/mL were detected for up to 4 weeks. Based on reported DTX clearance values in mice, these steady state plasma concentrations correspond to an *in vivo* release of approximately 5% per day (1.3 mg/kg/day), which is consistent with the above mentioned *in vitro* results. ALT levels in mice injected with DTX-loaded formulation were within acceptable limits, ranging from 12.8 to 30.8 U/L over 4 weeks (n = 11). Average ALT level in mice injected with the drug-free formulation was 17.1 $\pm$ 5.2 U/L (n = 11) over 7 weeks.

This novel injectable system provided sustained release of DTX *in vitro* and *in vivo* with demonstrated *in vivo* biocompatibility. These studies indicate the potential use of this injectable blend for localized sustained intraperitoneal release of DTX in the treatment of disease, for example, advanced ovarian cancer.

Components of Drug-free and Drug-loaded Blend Formulation

The drug-free blend formulation consisted of water-soluble high molecular weight chitosan, egg phosphatidylcholine and laurinaldehyde in a 1:4:1 weight ratio. The drug-loaded blend formulation consisted of 30 mg of DTX per gram of blend formulation to create a final drug to matrix ratio of 1:8 (w/w).

*In Vitro* Release of Docetaxel from Blend Formulation

*In vitro* release studies were performed to confirm the release profile of DTX from the blend formulation. This was the same formulation used for the *in vivo* studies. 0.5μL (0.450g) of the DTX-loaded blend formulation (1:8 drug: matrix, n = 6) was incubated at 37°C in 0.01 M phosphate-buffer solution (PBS) and 2 mg/mL lysozyme. At selected times, 2 mL aliquots were analyzed for DTX by HPLC. Drug-free blend formulations were also incubated in PBS/lysozyme and used as controls.

Plasma from control and treatment groups were analyzed for DTX by HPLC to determine *in vivo* release rate of drug from the blend formulation. *In vitro* release of $^3$H-DTX was shown to be 4.6% per day (control, n = 1, treatment, n=3) over 21 days.

*In Vivo* Studies

Six to eight week old, healthy female CD-1 mice were intraperitoneally-injected with 30μL of the drug-free blend formulation (control group, n=1) or 30μL of the DTX-loaded blend formulation (1:8 drug:matrix, treatment group, n = 3).

The mice in the treatment group received a DTX dose of 8 mg/kg.

Mice were assessed for the following biocompatibility endpoints: weight loss; post mortem examination for local inflammation and capsid formation; ALT measurements on plasma to assess liver toxicity; and hematoxylin and eosin staining on kidney, spleen, liver, and intestines.

*In vivo* biocompatibility of drug-free and drug-loaded blends was confirmed in the mice. Prior to sacrificing, mice appeared healthy without significant weight loss. Post-mortem examination did not show local peritoneal inflammation. Minimal to no capsid formation was observed.

ALT Levels in CD-1 Mice

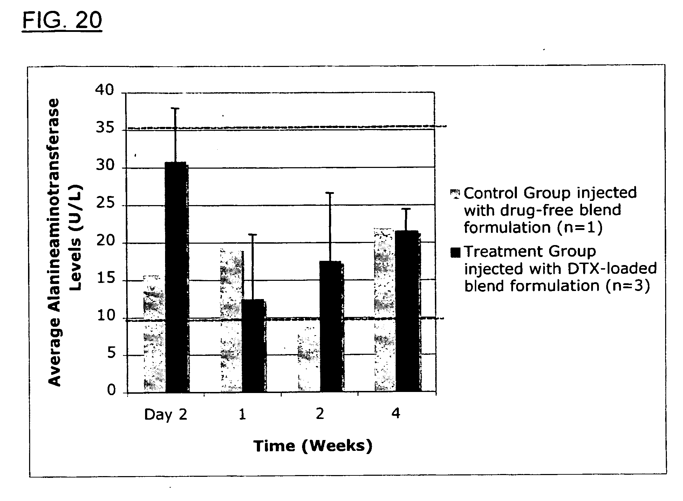

As shown in Fig. 20, ALT levels of treated mice injected with DTX-loaded formulation were within acceptable limits (10 - 35 units/L) ranging from 12.8 to 30.8 U/L over 4 weeks. The average ALT level in the treated mice was 20.6 $\pm$ 7.7 U/L (n=11). Average ALT level in the control mice, injected with the drug-free formulation, was 17.1 $\pm$ 5.2 U/L (n = 11) over 7 weeks.

Plasma Concentration Levels of Docetaxel in Mice

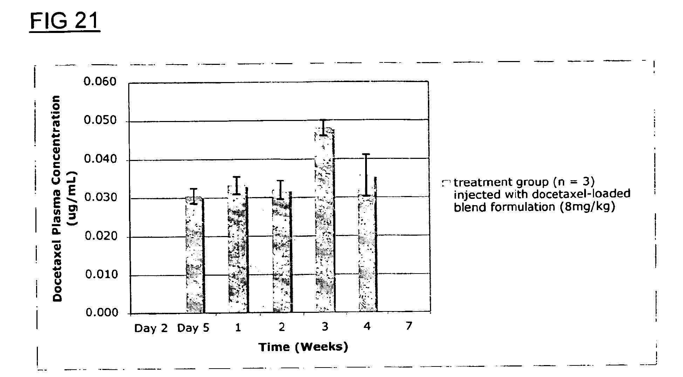

Figure 21 depicts DTX plasma levels. DTX plasma levels of 0.033 $\pm$ 0.01 ug/mL were detected for up to 4 weeks. Based on reported DTX clearance values in mice, these steady state plasma concentrations corresponded to an *in vivo* release of approximately 5% per day (1.3 mg/kg/day), which is consistent with *in vitro* findings. No detectable amounts of docetaxel were found in mice sacrificed (n=3) at Day 2 and at Week 7.

**Claims**

1. An injectable composition comprising a semi-solid blend of:

   (a) a chitosan based material;
   (b) a fatty acid or a fatty acid chloride or a fatty aldehyde, having an acyl chain length of C8-C16;
   (c) a phospholipid; and
   (d) at least one pharmaceutically active agent.

2. The injectable composition of claim 1 wherein the chitosan based material is water soluble chitosan.

3. The injectable composition of claim 2 wherein the water soluble chitosan is prepared by conjugating a chitosan-based material with glycidyltrimethylammonium chloride.

4. The injectable composition of claim 1 wherein the fatty acid has an acyl chain length of C10-C14.

5. The injectable composition of claim 1 wherein the phospholipid is selected from the group consisting of phosphatidylcholines, phosphatidylethanolamine, ePC and phosphatidylglycerol.

6. The injectable composition of claim 1 wherein the chitosan based material is water soluble chitosan, the fatty acid has an acyl chain length of C 12, and the phospholipid is selected from the group consisting of phosphatidylcholines, phosphatidylethanolamine, ePC and phosphatidylglycerol.

7. The injectable composition according to any one of claims 1 to 6 wherein the fatty acid is selected from the group consisting of a fatty aldehyde and a fatty acid chloride.

8. The injectable composition of claim 7, wherein the fatty aldehyde is laurinaldehyde.

9. The injectable composition of any one of claims 1 to 8, wherein the at least one pharmaceutically active agent is selected from the group consisting of carmustine, methotrexate, carboplatin, cisplatin, oxaliplatin, 5-fluorouracil, 5-fluorouridine, cytarabine, leuprolide acetate, cyclophosphamide, vinorelbine, pilocarpine, paclitaxel, mitomycin, camptothecin, doxorubicin, daunorubicin and docetaxel.

10. The injectable composition of any one of claims 1 to 8, wherein the at least one pharmaceutically active agent is selected from the group consisting of oligonucleotide, peptide and protein.

**11.** A drug delivery platform for the loading, delivery and controlled release of a pharmaceutically active agent, the drug delivery platform comprising:

(a) a water soluble chitosan;
(b) a fatty acid having an acyl chain length of C12; and
(c) a phospholipid selected from the group consisting of phosphatidylcholines, phosphatidylethanolamine, ePC and phosphatidylglycerol.

**12.** An injectable drug delivery composition comprising:

(a) a chitosan based material;
(b) a fatty acid having an acyl chain length of C8-C 16;
(c) a phospholipid; and
(d) at least one pharmaceutically active agent;

for use in the treatment or prevention of a disease in a mammal.

**13.** The injectable composition according to claim 12 wherein the disease is a cancer.

**14.** A method of manufacturing the injectable composition of claim 2 comprising the following steps:

(a) preparing water soluble chitosan from a chitosan-based material; and
(b) forming a complex of the water soluble chitosan with the fatty acid and the phospholipid to form an injectable material.


**Patentansprüche**

**1.** Injizierbare Zusammensetzung, welche eine halbfeste Mischung aus Folgendem umfasst:

(a) ein Material auf Chitosan-Basis;
(b) eine Fettsäure oder ein Fettsäurechlorid oder ein Fettaldehyd mit einer Acyl-Kettenlänge von C8-C16;
(c) ein Phospholipid; und
(d) mindestens einen pharmazeutischen Wirkstoff.

**2.** Injizierbare Zusammensetzung nach Anspruch 1, wobei das Material auf Chitosan-Basis wasserlösliches Chitosan ist.

**3.** Injizierbare Zusammensetzung nach Anspruch 2, wobei das wasserlösliche Chitosan durch Konjugieren eines Chitosan-basierten Materials mit Glycidyltrimethylammoniumchlorid hergestellt wird.

**4.** Injizierbare Zusammensetzung nach Anspruch 1, wobei die Fettsäure eine Acyl-Kettenlänge von C10-C14 aufweist.

**5.** Injizierbare Zusammensetzung nach Anspruch 1, wobei das Phospholipid ausgewählt ist aus der Gruppe bestehend aus Phosphatidylcholinen, Phosphatidylethanolamin, ePC und Phosphatidylglycerin.

**6.** Injizierbare Zusammensetzung nach Anspruch 1, wobei das Material auf Chitosan-Basis wasserlösliches Chitosan ist, die Fettsäure eine Acyl-Kettenlänge von C12 aufweist und das Phospholipid ausgewählt ist aus der Gruppe bestehend aus Phosphatidylcholinen, Phosphatidylethanolamin, ePC und Phosphatidylglycerin.

**7.** Injizierbare Zusammensetzung nach einem der Ansprüche 1 bis 6, wobei die Fettsäure ausgewählt ist aus der Gruppe bestehend aus einem Fettaldehyd und einem Fettsäurechlorid.

**8.** Injizierbare Zusammensetzung nach Anspruch 7, wobei das Fettaldehyd Laurinaldehyd ist.

**9.** Injizierbare Zusammensetzung nach einem der Ansprüche 1 bis 8, wobei der mindestens eine pharmazeutische Wirkstoff ausgewählt ist aus der Gruppe bestehend aus Carmustin, Methotrexat, Carboplatin, Cisplatin, Oxaliplatin, 5-Fluoruracil, 5-Fluoruridin, Cytarabin, Leuprolidacetat, Cyclophosphamid, Vinorelbin, Pilocarpin, Paclitaxel, Mitomycin, Camptothecin, Doxorubicin, Daunorubicin und Docetaxel.

10. Injizierbare Zusammensetzung nach einem der Ansprüche 1 bis 8, wobei der mindestens eine pharmazeutische Wirkstoff ausgewählt ist aus der Gruppe bestehend aus Oligonukleotid, Peptid und Protein.

11. Arzneimittelbereitstellungsplattform zum Laden, Bereitstellen und kontrollierten Freisetzen eines pharmazeutischen Wirkstoffes, wobei die Arzneimittelbereitstellungsplattform Folgendes umfasst:

   (a) ein wasserlösliches Chitosan;
   (b) eine Fettsäure mit einer Acyl-Kettenlänge von C12; und
   (c) ein Phospholipid ausgewählt aus der Gruppe bestehend aus Phosphatidylcholinen, Phosphatidylethanolamin, ePC und Phosphatidylglycerin.

12. Injizierbare Arzneimittelbereitstellungszusammensetzung, welche Folgendes umfasst:

   (a) ein Material auf Chitosan-Basis;
   (b) eine Fettsäure mit einer Acyl-Kettenlänge von C8-C16;
   (c) ein Phospholipid; und
   (d) mindestens einen pharmazeutischen Wirkstoff;

zur Verwendung bei der Behandlung oder Prävention einer Erkrankung bei einem Säugetier.

13. Injizierbare Zusammensetzung nach Anspruch 12, wobei die Erkrankung ein Krebs ist.

14. Verfahren zur Herstellung der injizierbaren Zusammensetzung nach Anspruch 2, welches folgende Schritte enthält:

   (a) Herstellen von wasserlöslichem Chitosan aus einem Chitosan-basierten Material; und
   (b) Bilden eines Komplexes aus dem wasserlöslichen Chitosan mit der Fettsäure und dem Phospholipid zum Bilden eines injizierbaren Materials.

**Revendications**

1. Composition injectable comprenant un mélange semi-solide de :

   (a) un matériau à base de chitosan ;
   (b) un acide gras ou un chlorure d'acide gras ou un aldéhyde gras, ayant une longueur de chaîne acyle de C8-C16 ;
   (c) un phospholipide ; et
   (d) au moins un agent pharmaceutiquement actif.

2. Composition injectable selon la revendication 1, dans laquelle le matériau à base de chitosan est du chitosan soluble dans l'eau.

3. Composition injectable selon la revendication 2, dans laquelle le chitosan soluble dans l'eau est préparé par conjugaison d'un matériau à base de chitosan et de chlorure de glycidyltriméthylammonium.

4. Composition injectable selon la revendication 1, dans laquelle l'acide gras a une longueur de chaîne acyle de C10-C14.

5. Composition injectable selon la revendication 1, dans laquelle le phospholipide est choisi dans le groupe constitué des phosphatidylcholines, de la phosphatidyléthanolamine, de l'ePC et du phosphatidylglycérol.

6. Composition injectable selon la revendication 1, dans laquelle le matériau à base de chitosan est du chitosan soluble dans l'eau, l'acide gras a une longueur de chaîne acyle de C12 et le phospholipide est choisi dans le groupe constitué des phosphatidylcholines, de la phosphatidyléthanolamine, de l'ePC et du phosphatidylglycérol.

7. Composition injectable selon l'une quelconque des revendications 1 à 6, dans laquelle l'acide gras est choisi dans le groupe constitué d'un aldéhyde gras et d'un chlorure d'acide gras.

8. Composition injectable selon la revendication 7, dans laquelle l'aldéhyde gras est le laurinaldéhyde.

9. Composition injectable selon l'une quelconque des revendications 1 à 8, dans laquelle le au moins un agent pharmaceutiquement actif est choisi dans le groupe constitué de la carmustine, du méthotrexate, de la carboplatine, de la cisplatine, de l'oxaliplatine, du 5-fluorouracil, de la 5-fluorouridine, de la cytarabine, de l'acétate de leuprolide, du cyclophosphamide, de la vinorelbine, de la pilocarpine, du paclitaxel, de la mitomycine, de la camptothécine, de la doxorubicine, de la daunorubicine et du docétaxel.

10. Composition injectable selon l'une quelconque des revendications 1 à 8, dans laquelle le au moins un agent pharmaceutiquement actif est choisi dans le groupe constitué d'un oligonucléotide, d'un peptide et d'une protéine.

11. Plate-forme de délivrance d'un médicament pour charger, délivrer ou libérer de façon contrôlée un agent pharmaceutiquement actif, la plate-forme de délivrance d'un médicament comprenant :

   (a) du chitosan soluble dans l'eau ;
   (b) un acide gras ayant une longueur de chaîne acyle de C12 ; et
   (c) un phospholipide est choisi dans le groupe constitué des phosphatidylcholines, de la phosphatidyléthanolamine, de l'ePC et du phosphatidylglycérol.

12. Composition injectable de délivrance d'un médicament comprenant :

   (a) un matériau à base de chitosan ;
   (b) un acide gras ayant une longueur de chaîne acyle de C8-C16 ;
   (c) un phospholipide ; et
   (d) au moins un agent pharmaceutiquement actif ;

   destinée à une utilisation dans le traitement ou la prévention d'une maladie chez un mammifère.

13. Composition injectable selon la revendication 12, dans laquelle la maladie est un cancer.

14. Procédé de fabrication de la composition injectable selon la revendication 2, comprenant les étapes suivantes :

   (a) la préparation de chitosan soluble dans l'eau à partir d'un matériau à base de chitosan ; et
   (b) la formation d'un complexe du chitosan soluble dans l'eau avec l'acide gras et le phospholipide pour former un matériau injectable.

WATER SOLUBLE CHITOSAN (WSC)

+

EGG PHOSPHATIDYLCHOLINE (ePC)

+

FATTY ACID CHLORIDE (X=Cl) OR FATTY ALDEHYDE (X=H)

**FIG. 1**

FIG. 2

(a)

(b)

(c)

**FIG. 3**

**FIG. 4**

(b)

FIG. 4

(a)

(b)

(c)

**FIG. 5**

(a)  (b)  (c)

**FIG. 6**

(a)

(b)

FIG. 7

**FIG. 8**

(a)

(b)

**FIG. 9**

**FIG. 10**

**FIG. 11**

**FIG. 12**

FIG. 13

FIG. 14

**FIG. 15**

**FIG. 16**

**FIG. 17**

**FIG. 18**

**FIG. 19A**

**FIG. 19B**

FIG. 20

## FIG 21

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20030134810 A
- US 7060285 B, Muller

- US 20050208122 A, Allen

**Non-patent literature cited in the description**

- **CHO, J. et al.** *Biomacromolecules,* 2006, vol. 7 (10), 2845-2855
- **LE TIEN, C. et al.** *Journal of Controlled Release,* 2003, vol. 93 (1), 1-13

- **HOFFMAN.** *J. Control. Rel.,* 2001, vol. 72, 35-46
- **SEONG, H.S ; H.S. WHANG ; S.W. KO.** *Journal of Applied Polymer Science,* 2000, vol. 76 (14), 2009-2015